(19) Europäisches Patentamt
European Patent Office
Office européen des brevets

(11) EP 0 624 586 B1

(12) # EUROPEAN PATENT SPECIFICATION

(45) Date of publication and mention of the grant of the patent: **01.09.1999 Bulletin 1999/35**

(21) Application number: **94106812.4**

(22) Date of filing: **02.05.1994**

(51) Int. Cl.$^6$: **C07D 471/04**, C07D 487/04, A61K 31/55 // (C07D471/04, 221:00, 209:00), (C07D487/04, 223:00, 209:00), (C07D487/04, 209:00, 209:00)

(54) **Substituted pyrroles**

Substituierte Pyrrole

Pyroles substituées

(84) Designated Contracting States:
**AT BE CH DE DK ES FR GB GR IE IT LI LU MC NL PT SE**
Designated Extension States:
**SI**

(30) Priority: **10.05.1993 GB 9309602**
**21.02.1994 GB 9403249**

(43) Date of publication of application:
**17.11.1994 Bulletin 1994/46**

(73) Proprietor:
**F. HOFFMANN-LA ROCHE AG**
**4002 Basel (CH)**

(72) Inventors:
- **Harris, William**
  **Welwyn, Herts. (GB)**
- **Hill, Christopher Huw**
  **Knebworth, Herts. (GB)**
- **Lawton, Geoffrey**
  **St. Ippollitts, Hitchin, Herts. (GB)**

(74) Representative: **Mahé, Jean et al**
**F.Hoffmann-La Roche AG**
**Patent Department (PLP),**
**124 Grenzacherstrasse**
**4070 Basel (CH)**

(56) References cited:
**EP-A- 0 328 000**
**EP-A- 0 328 026**
**EP-A- 0 362 695**
**EP-A- 0 384 349**
**EP-A- 0 470 490**

- **CHEMICAL ABSTRACTS, vol. 118, no. 5, 1 February 1993, Columbus, Ohio, US; abstract no. 38722e, & J. MED. CHEM., vol.36, no.1, 1993, WASHINGTON US pages 21 - 29 R.A. BIT ET AL.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 624 586 B1

## Description

The present invention relates to substituted pyrroles. More particularly, the invention is concerned with compounds of the general formula I

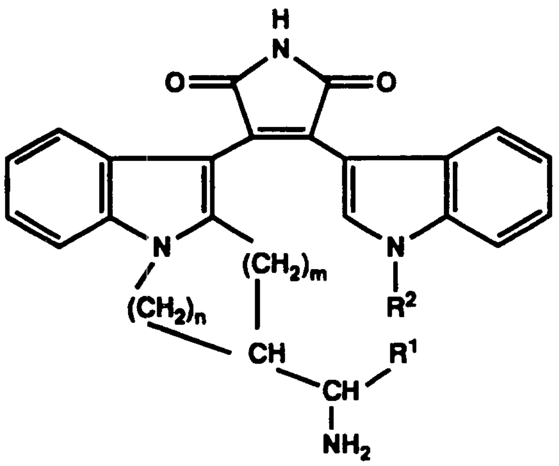

I

wherein $R^1$ represents lower alkyl, lower cycloalkyl, aryl or lower aralkyl, $R^2$ represents hydrogen, aryl or lower alkyl optionally substituted by hydroxy, acyloxy, amino, mono(lower alkyl)amino, di(lower alkyl)amino, carboxy, lower alkoxycarbonyl or aminocarbonyl and m and n stand for 1 or 2,

as well as pharmaceutically acceptable salts of acidic compounds of formula I with bases and of basic compounds of formula I with acids.

Objects of the present invention are the compounds of formula I and their aforementioned salts per se and as therapeutically active substances; a process for the manufacture of said compounds and salts and novel intermediates useful in said process; medicaments containing said compounds and salts and the manufacture of these medicaments; and the use of said compounds and salts in the control or prevention of illnesses, especially in the control or prevention of inflammatory, immunological, oncological, bronchopulmonary, dermatological and cardiovascular disorders, in the treatment of asthma, AIDS or diabetic complications or for the stimulation of hair growth, or for the manufacture of a medicament against inflammatory, immunological, oncological, bronchopulmonary, dermatological and cardiovascular disorders or against asthma, AIDS or diabetic complications or for the stimulation of hair growth.

EP-A-0 384 349 discloses various bisindolyl maleimedes which basically contain an additional tetrahydropyrido, tertahydroazepino or tetrahydropyrazino moiety which can be substituted in one position of said moiety.

Bit et al., J. Med. Chem. 36, 1, 21-29 (1993), describe the chemical synthesis of bisindolyl maleimides having an additional tetrahydropyrrolo, tetrahydroazepino or tetrahydropyrido ring substituted with an aminoalkyl group.

EP-A-0 470 490 discloses bisindolyl maleimides having an additional cycloalkyl ring which is further substituted with a cycloalkyl or aza-cycloalkyl group.

The compounds of the present invention differ from the prior art compounds in having at least a different substituent attached to the tetrahydropyrrolo, -pyrido or -azepino ring.

As used herein, the term "lower alkyl", alone or in combination, means a straight-chain or branched-chain alkyl group containing 1-6 carbon atoms such as methyl, ethyl, propyl, isopropyl, butyl, sec.butyl, tert.butyl, pentyl and the like. The term "lower alkoxy", alone or in combination, means an alkyl group as defined earlier which is attached via an oxygen atom, examples of alkoxy groups being methoxy, ethoxy, propoxy, isopropoxy, butoxy, tert.butoxy and the like. The term "lower cycloalkyl" means a cycloalkyl group containing 3-6 carbon atoms, i.e. cyclopropyl, cyclobutyl, cyclopentyl and cyclohexyl. The term "aryl" means unsubstituted phenyl or phenyl carrying one or more substituents selected from e.g. halogen, lower alkyl and lower alkoxy, such as p-chlorophenyl, p-tolyl and p-methoxyphenyl. The term "lower aralkyl" means a lower alkyl group as hereinbefore defined in which one hydrogen atom has been replaced by an aryl group as hereinbefore defined, such as benzyl, 2-phenylethyl, p-chlorobenzyl, p-methylbenzyl and p-methoxybenzyl. The term "acyloxy" means an acyloxy group derived from an alkanoic acid containing up to 6 carbon atoms, e.g. acetoxy, propionyloxy or butyryloxy, or from an aromatic carboxylic acid which can be optionally substituted by e.g. halogen, lower alkyl and/or lower alkoxy, e.g. benzoyloxy, p-chlorobenxoyloxy, p-toluoyloxy and p-methoxybenzoyloxy. The term "halogen" means fluorine, chlorine, bromine or iodine.

The compounds of formula I contain two chiral carbon atoms and can therefore be present in racemic or optically active forms. The present invention includes within its scope not only the racemic compounds, but also the optically active isomers.

In the compounds of formula I, $R^1$ preferably represents lower alkyl, especially lower alkyl containing 1-3 carbon atoms. $R^2$ preferably represents lower alkyl, especially methyl. Preferably, m stands for 1 and n stands for 2.

Especially preferred compounds of formula I hereinbefore are:

3-[8(S)-[1(R or S)-Aminopropyl]-6,7,8,9-tetrahydropyrido[1,2-a]indol-10-yl]-4-(1-methyl-3-indolyl)-1H-pyrrole-2,5-dione and
3-[8(S)-[1(S)-amino-2-methylpropyl]-6,7,8,9-tetrahydropyrido[1,2-a]indol-10-yl]-4-(1-methyl-3-indolyl)-1H-pyrrole-2,5-dione.

Other preferred compounds of formula I are:

3-[8(R or S)-1(R or S)-Aminoethyl]-6,7,8,9-tetrahydropyrido[1,2-a]indol-10-yl]-4-(1-methyl-3-indolyl)-1H-pyrrole-2,5-dione,
3-[8(R or S)-1(R or S)-aminopropyl]-6,7,8,9-tetrahydropyrido[1,2-a]indol-10-yl]-4-(1-methyl-3-indolyl)-1H-pyrrole-2,5-dione,
3-[8(R or S)-1(R or S)-aminobutyl]-6,7,8,9-tetrahydropyrido[1,2-a]indol-10-yl]-4-(1-methyl-3-indolyl)-1H-pyrrole-2,5-dione and
3-[8(R or S)-1(R or S)-amino-2-methylpropyl]-6,7,8,9-tetrahydropyrido[1,2-a]indol-10-yl]-4-(1-methyl-3-indolyl)-1H-pyrrole-2,5-dione.

The following are also preferred compounds falling under formula I:

3-[8(S)-1(R)-Amino-2-methylpropyl]-6,7,8,9-tetrahydropyrido[1,2-a]indol-10-yl]-4-(1-methyl-3-indolyl)-1H-pyrrole-2,5-dione,
3-[8(R or S)-[alpha (R or S)-aminobenzyl]-6,7,8,9-tetrahydropyrido[1,2-a]indol-10-yl]-4-(1-methyl-3-indolyl)-1H-pyrrole-2,5-dione,
3-[8(S)-[(R or S)-(amino)(cyclopentyl)methyl-6,7,8,9-tetrahydropyrido[1,2-a]indol-10-yl]-4-(1-methyl-3-indolyl)-1H-pyrrole-2,5-dione,
3-[2(R or S)-[1(R or S)-amino-2-methylpropyl]-2,3-dihydro-1H-pyrrolo[1,2-a]indol-9-yl]-4-(1-methyl-3-indolyl)-1H-pyrrole-2,5-dione,
3-[8(RS)-[1(RS)-amino-2-methylpropyl]-7,8,9,10-tetrahydro-6H-azepino[1,2-a]indol-11-yl]-4-(1-methyl-3-indolyl)-1H-pyrrole-2,5-dione,
3-[7(RS)-[1(RS)-amino-2-methylpropyl]-6,7,8,9-tetrahydropyrido[1,2-a]indol-9-yl]-4-(1-methyl-3-indolyl)-1H-pyrrole-2,5-dione and
3-[8(S)-[1(S)-amino-2-methylpropyl]-6,7,8,9-tetrahydropyrido[1,2-a]indol-10-yl]-4-(1-phenyl-3-indolyl)-1H-pyrrole-2,5-dione.

According to the process provided by the present invention, the compounds of formula I as well as pharmaceutically acceptable salts of acidic compounds of formula I with bases and of basic compounds of formula I with acids are manufactured by cleaving off the protecting group denoted by $R^3$ from a compound of the general formula

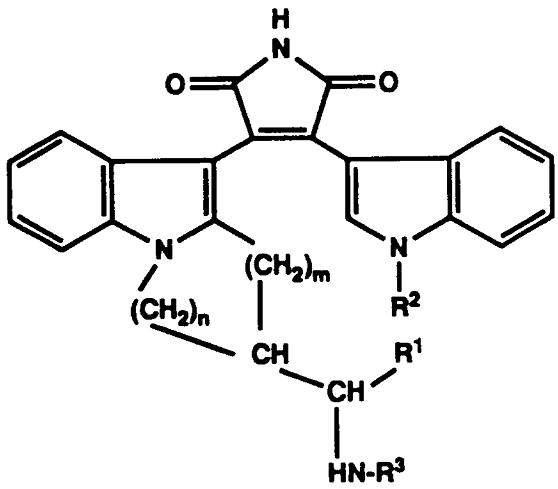

II

wherein $R^1$, $R^2$, m and n have the significance given earlier and $R^3$ represents a urethane protecting group, and, if desired, functionally modifying a reactive substituent present in $R^2$ in a compound of formula I obtained and, also if desired, converting an acidic compound of formula I into a pharmaceutically acceptable salt with a base or converting a basic compound of formula I into a pharmaceutically acceptable salt with an acid.

The urethane protecting group denoted by $R^3$ in formula II is preferably lower alkoxycarbonyl, especially tert.butoxycarbonyl, or lower aralkoxycarbonyl, especially benzyloxycarbonyl.

The cleavage of the protecting group denoted by $R^3$ from a compound of formula II can be carried out in a manner known per se. For example, when $R^3$ represents lower alkoxycarbonyl the cleavage can be carried out using a mineral acid such as hydrochloric acid in an inert organic solvent such as a cyclic ether, e.g. tetrahydrofuran or dioxan, an alkanol, e.g. methanol or ethanol, esters such as ethyl acetate or a halogenated, especially chlorinated, hydrocarbon, e.g. dichloromethane, or using trifluoroacetic acid. When $R^3$ represents an aralkoxycarbonyl group the cleavage is carried out by hydrogenolysis in a manner known per se; for example using hydrogen in the presence of a catalyst such as palladium/charcoal.

The functional modification of a reactive substituent present in $R^2$ in a compound of formula I obtained can comprise the esterification of a carboxy group to a lower alkoxycarbonyl group, the hydrolysis of an acyloxy group to a hydroxy group or the conversion of a lower alkoxycarbonyl group into a carboxy group. All of these modifications can be carried out according to methods which are known per se.

The conversion of an acidic compound of formula I into a pharmaceutically acceptable salt can be carried out by treatment with a suitable base in a manner known per se. Suitable salts are those derived not only from inorganic bases, for example, sodium salts, potassium salts, calcium salts and the like, but also from organic bases, for example ethylenediamine, monoethanolamine, diethanolamine and like salts. The conversion of a basic compound of formula I into a pharmaceutically acceptable salt can be carried out by treatment with a suitable acid in a manner known per se. Suitable salts are those derived not only from inorganic acids, for example, hydrochlorides, hydrobromides, phosphates, sulphates and the like, but also from organic acids, for example acetates, citrates, fumarates, tartrates, maleates, methanesulphonates, p-toluenesulphonates and the like.

The starting materials of formula II hereinbefore are novel and also form an object of the present invention. They can be prepared, for example, by:

(a) reacting a compound of the general formula

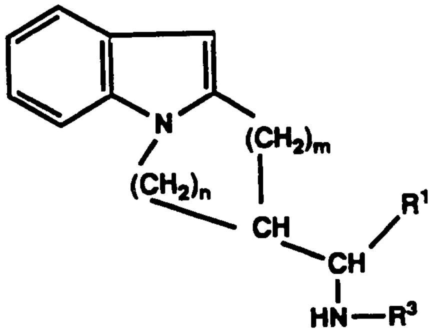

III

wherein $R^1$, $R^3$, m and n have the significance given earlier, with oxalyl chloride, condensing the resulting activated glyoxylate of the general formula

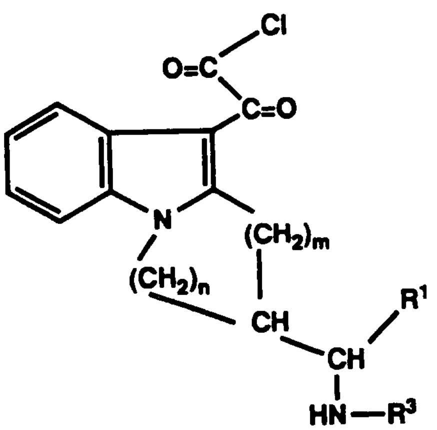

IV

wherein $R^1$, $R^3$, m and n have the significance given earlier, with an imidate of the general formula

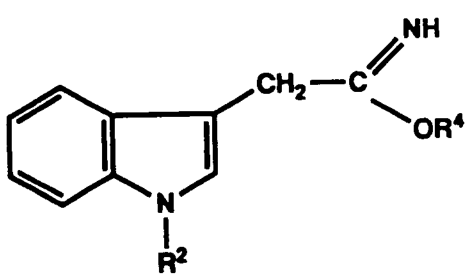

V

wherein $R^2$ has the significance given earlier and $R^4$ represents lower alkyl, in the presence of a strong base and hydrolyzing and dehydrating the resulting hydroxy-pyrrolinone of the general formula

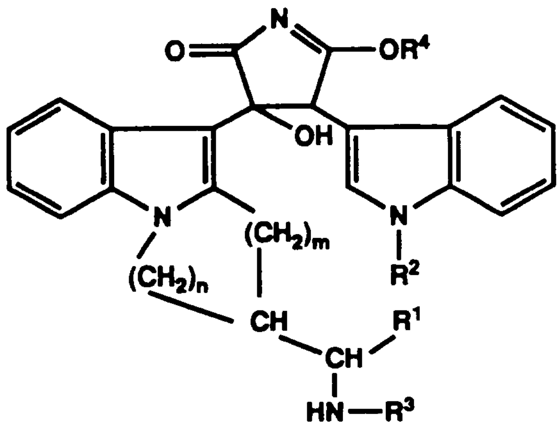

VI

wherein $R^1$, $R^2$, $R^3$, $R^4$, m and n have the significance given earlier,

or

(b) reacting an activated glyoxylate of formula IV hereinbefore with an indolylacetic acid of the general formula

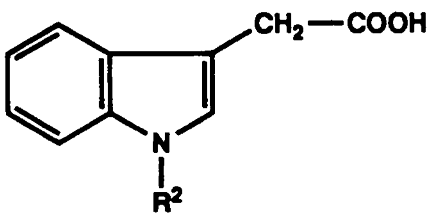

VII

wherein $R^2$ has the significance given earlier,

in the presence of a strong base and converting the resulting substituted furandione of the general formula

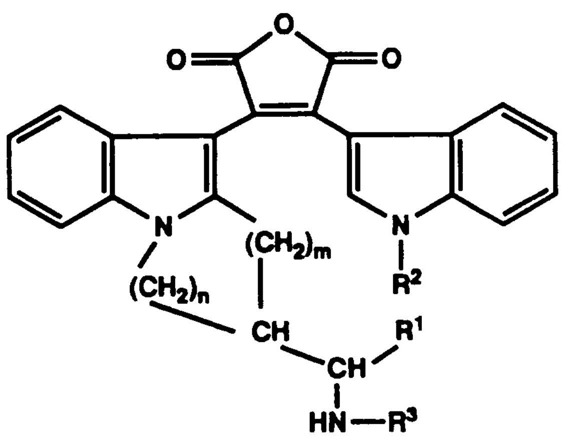

VIII

wherein $R^1$, $R^2$, $R^3$, m and n have the significance given earlier, into the corresponding imide starting material of formula II.

The reaction of a compound of formula III with oxalyl chloride is conveniently carried out in the presence of an inert organic solvent, suitably a halogenated aliphatic hydrocarbon such as dichloromethane. It is also convenient to carry out this reaction at about 0°C.

The condensation of an activated glyoxylate of formula IV with an imidate of formula V, which is a known compound or an analogue of a known compound, is conveniently carried out in an inert organic solvent. Suitable bases are,

for example, tertiary amines such as triethylamine, diisopropylethylamine, 4-dimethylaminopyridine, N-ethylmorpholine and 1,4-diazabicyclo[2.2.2]octane as well as pyridine. Suitable solvents are, for example, halogenated aliphatic hydrocarbons such as dichloromethane and chloroform, optionally halogenated aromatic hydrocarbons such as benzene, toluene and chlorobenzene, open-chain and cyclic ethers such as dimethoxyethane, tert.butyl methyl ether and tetrahydrofuran, formamides such as dimethylformamide, esters such as ethyl acetate and nitriles such as acetonitrile. The condensation is preferably carried out at about 0°C to 40°C, especially at room temperature. Further, it is preferred to carry out this condensation in situ.

The hydrolysis and dehydration of a hydroxy-pyrrolinone of formula VI to give a compound of formula II is expediently carried out by treatment with a mineral acid such as hydrochloric acid or sulphuric acid or an organic acid such as methanesulphonic acid or p-toluenesulphonic acid or by treatment with an acylating reagent such as trifluoroacetic anhydride and a suitable base such as pyridine, conveniently at about room temperature. The hydroxypyrrolinone of formula VI is preferably hydrolyzed and dehydrated in situ.

The reaction of an activated glyoxylate of formula IV with an indolylacetic acid of formula VII, which is a known compound or an analogue of a known compound, is conveniently carried out in a manner analogous to that described earlier in connection with the condensation of an activated glyoxylate of formula IV with an imidate of formula V.

The conversion of a substituted furandione of formula VIII into a desired imide starting material of formula II can be carried out conveniently by treatment with hexamethyldisilazane in the presence of an alkanol such as methanol in an inert organic solvent. Suitable solvents are, for example, halogenated aliphatic hydrocarbons such as dichloromethane and chloroform, optionally halogenated aromatic hydrocarbons such as benzene, toluene and chlorobenzene, open-chain and cyclic ethers such as dimethoxyethane, tert.butyl methyl ether and tetrahydrofuran, or formamides such as dimethylformamide. The reaction is preferably carried out at about room temperature to 100°C, especially at about 50°C.

The compounds of formula III hereinbefore can be prepared, for example, as illustrated in Reaction Scheme I hereinafter in which $R^1$, m and n have the significance given earlier.

## Reaction Scheme I

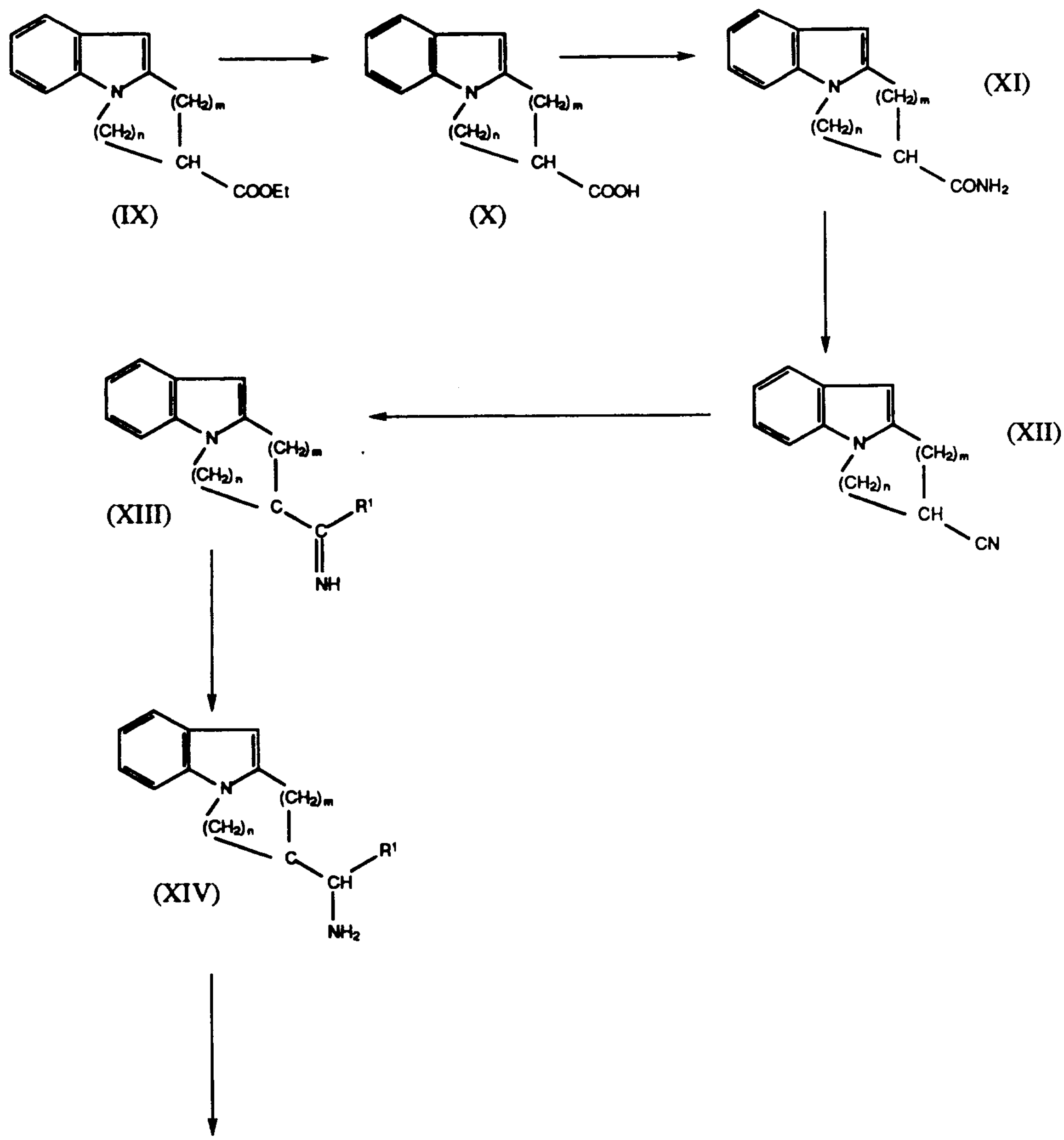

(IX) (X) (XI) (XII) (XIII) (XIV)

(III)

Having regard to Reaction Scheme I, all of the individual steps thereof can be carried out according to conventional methods. In the first step, an ethyl ester of formula IX, which is a known compound or an analogue of a known compound, is saponified to the corresponding acid of formula X using e.g. sodium hydroxide solution. The resulting acid is then amidated, e.g. by reaction with ethyl chloroformate in the presence of triethylamine followed by treatment with ammonia, and the resulting amide of formula XI is converted into the nitrile of formula XII using e.g. trifluoroacetic anhydride. Then, the nitrile of formula XII is reacted with a Grignard reagent of the formula $R^1$-Mg-X, wherein $R^1$ has the significance given earlier and X represents halogen, preferably chlorine, and the resulting imine of formula XIII is reduced using a complex metal hydride, e.g. lithium aluminium hydride, to a primary amine of formula XIV. This reduction is preferably carried out in situ. The primary amine of formula XIV is converted into a compound of formula III by, for example,

reaction with a chloroformate of the formula $R^3Cl$ or an anhydride of the formula $R^3OR^3$, wherein $R^3$ has the significance given earlier, in the presence of a base such as triethylamine.

Homochiral compounds of formula III, denoted hereinafter as IIIA, can be prepared, for example, as illustrated in the following Reaction Scheme II in which $R^1$, m and n have the significance given earlier and $R^5$ represents lower alkyl.

Reaction Scheme II

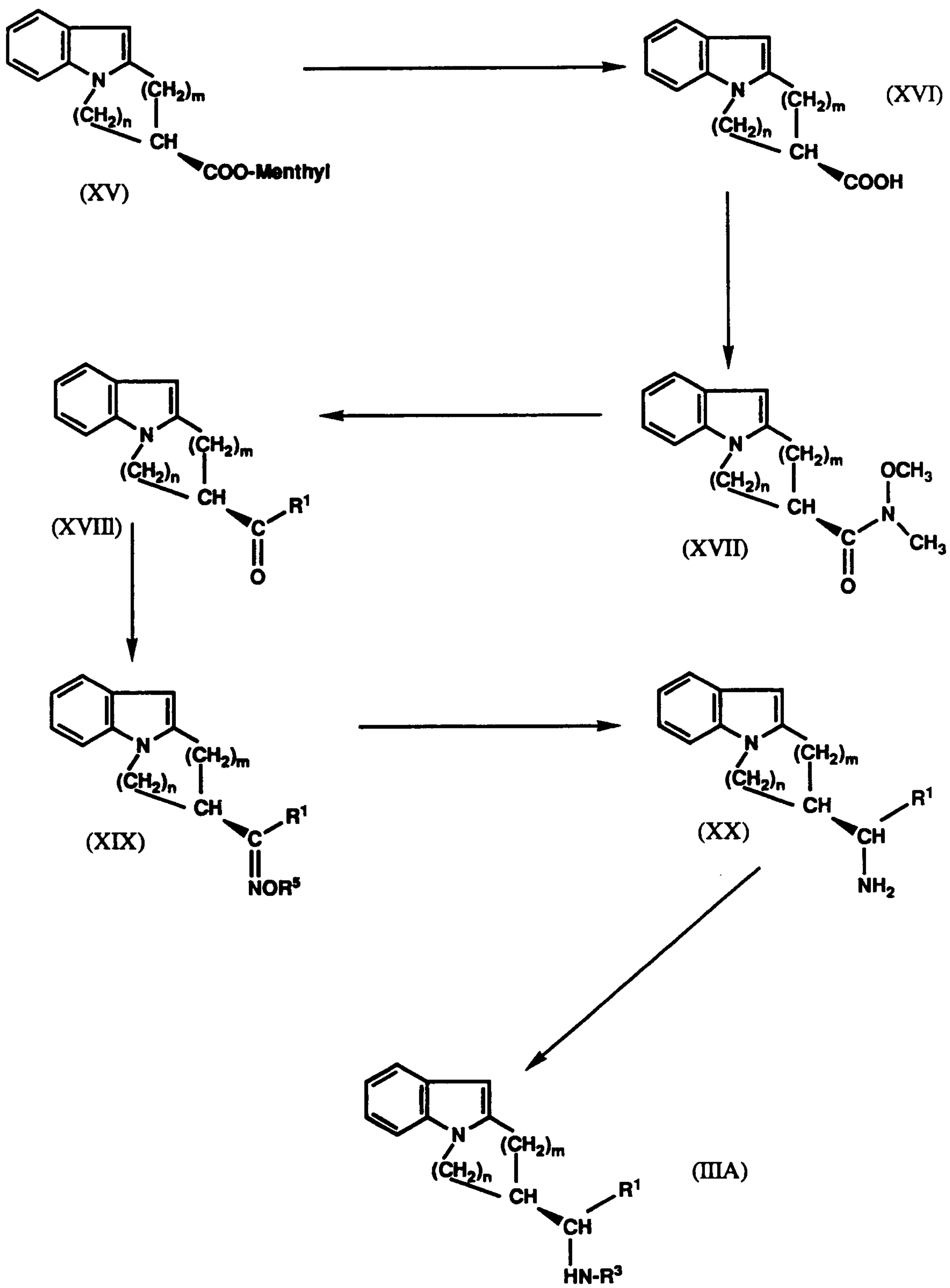

(XV) (XVI) (XVIII) (XVII) (XIX) (XX) (IIIA)

The individual steps of the synthesis illustrated in Reaction Scheme II can all be carried out according to methods known per se. In the first step, a menthyl ester of formula XV, which is a known compound or an analogue of a

known compound, is converted with a strong acid, e.g. concentrated sulphuric acid, into the corresponding carboxylic acid of formula XVI. This acid is then condensed with N,O-dimethylhydroxylamine and the N-methoxy-N-methyl carboxamide of formula XVII obtained is reacted with a Grignard reagent of the formula $R^1$-Mg-X, wherein $R^1$ has the significance given earlier and X represents halogen, preferably chlorine, to give a ketone of formula XVIII. Reaction of this ketone with a hydroxylamine of the formula $H_2N$-$OR^5$, wherein $R^5$ has the significance given earlier, gives an oxime of formula XIX which is reduced with a complex metal hydride such as lithium aluminium hydride to a primary amine of formula XX. The latter is subsequently converted into a homochiral starting material of formula IIIA by acylation in an analogous manner to that described in Reaction Scheme I for the conversion of a compound of formula XIV into a compound of formula III.

Reaction Scheme III hereinafter illustrates an alternative route to homochiral starting materials of formula IIIA. In this Reaction Scheme $R^1$, m and n have the significance given earlier and Ms represents methanesulphonyl.

<u>Reaction Scheme III</u>

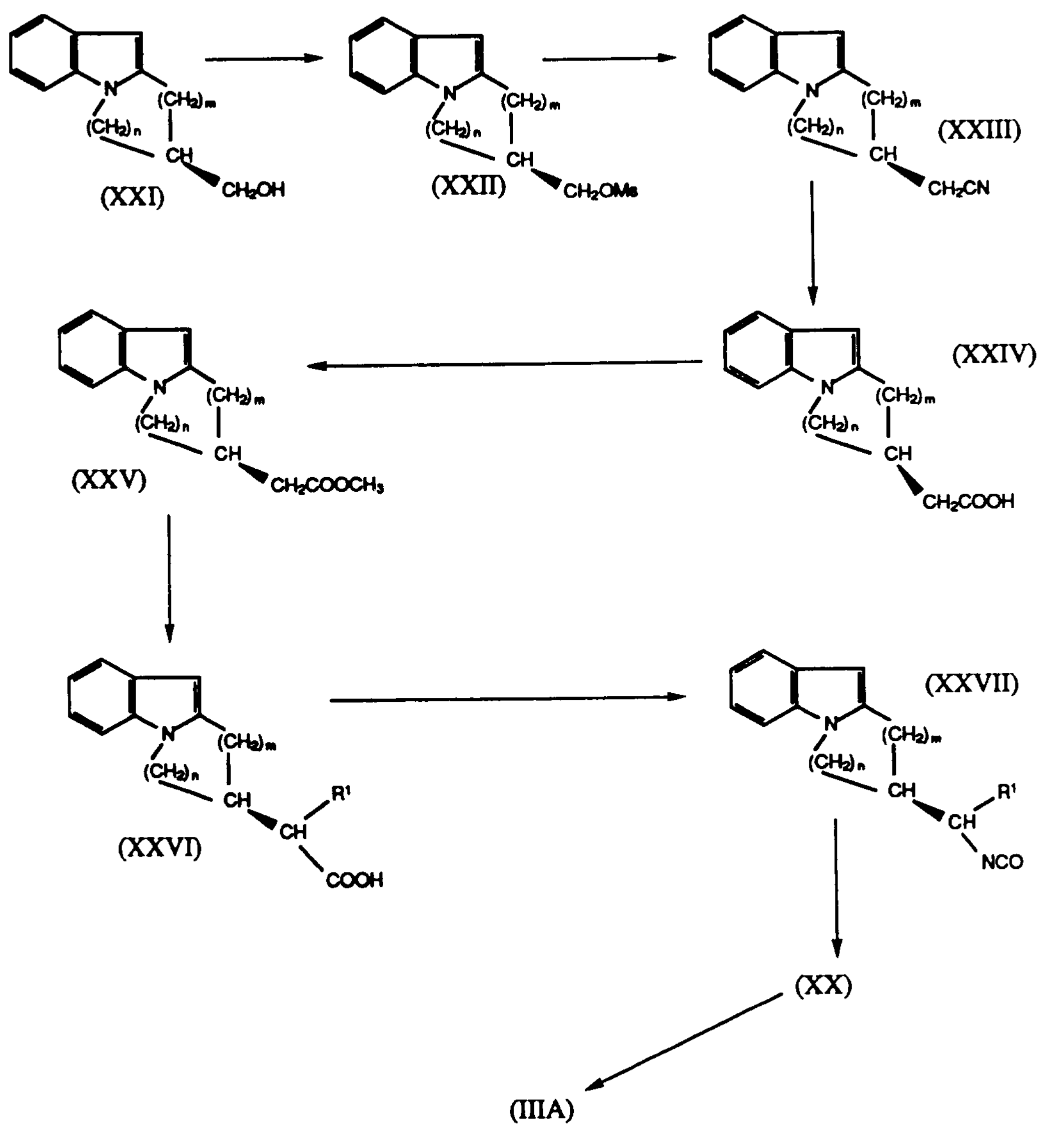

(XXI) (XXII) (XXIII) (XXIV) (XXV) (XXVI) (XXVII) (XX) (IIIA)

Having regard to Reaction Scheme III, all of the individual steps therein can be carried out in a conventional manner. Firstly, an alcohol of formula XXI is sulphonated, e.g. with methanesulphonic anhydride, and the methanesulphonate of formula XXII obtained is converted with sodium cyanide into the nitrile of formula XXIII. The latter is hydrolyzed, e.g. using sodium hydroxide solution, to the corresponding carboxylic acid of formula XXIV, which is methylated,

e.g. using methanol in the presence of concentrated sulphuric acid, to the methyl ester of formula XXV. Reaction of the latter with a halide of the formula $R^1$-X, wherein $R^1$ and X have the significance given earlier, in the presence of a strong base such as lithium diisopropylamide followed by treatment with sodium hydroxide solution yields the carboxylic acid of formula XXVI. The latter is reacted with diphenylphosphoryl azide to give the isocyanate of formula XXVII which is converted into a primary amine of formula XX by treatment with hydrochloric acid. The conversion of a primary amine of formula XX into a homochiral compound of formula IIIA is carried out by acylation in an analogous manner to that described in connection with Reaction Scheme I.

The compounds of formula I and their pharmaceutically acceptable salts are protein kinase inhibitors; they inhibit cellular processes, for example cell proliferation and secretion, and can be used in the control or prevention of illnesses, for example in the control or prevention of inflammatory disorders such as arthritis, immune diseases, psoriasis, contact dermatitis, in conjunction with organ transplants and also in oncology. They inhibit infection of cells with human immunodeficiency virus or Epstein-Barr virus and are thus useful in the treatment of AIDS and infectious mononucleosis. The compounds and salts of the present invention also inhibit smooth muscle contraction and can therefore be used against cardiovascular and bronchopulmonary disorders. Further, they are also useful in asthma therapy. The present compounds and salts also inhibit platelet aggregation and can be used in the control or prevention of thrombosis. Further, they inhibit the release of mediators from activated neutrophils and can therefore be used to control ischaemic damage, e.g. in the heart or brain. Again, they inhibit neurotoxicity induced by elevated glucose levels and are thus useful for the treatment of diabetic complications. Finally, the present compounds and salts stimulate hair growth and can therefore be used to prevent or repress baldness.

The activity of the present compounds in inhibiting protein kinase C can be demonstrated by means of the in vitro test procedure described hereinafter.

The assay system described by Takai et al., BBRC 19, 1218, (1979) is used. Reaction mixtures (100 µl) contain 10 µM [$\gamma$-$^{32}$P]ATP, 0.2 mg/ml (about 15 µM) lysine-rich histone, 0.5 mM $CaCl_2$ and 40 µg/ml phosphatidylserine in 25 mM Tris HCl, 5 mM $MgNO_3$ (pH 7.5) buffer. The enzyme protein kinase C is isolated from rat brains according to the method of Kikkawa et al., J. Biol. Chem. 257, 13341 (1982).

The reaction is started by adding the enzyme, is run at 30°C for 10 minutes and is then stopped using 1 ml of ice-cold 10% trichloroacetic acid. Acid-precipitable protein is collected on glass fibre discs by filtration. These discs are then washed with 5% trichloroacetic acid containing 20 mM sodium pyrophosphate (in order to remove unreacted ATP), followed by ethanol. The discs are dried and counted. Counts associated with each disc are used as a measure of the incorporation of $^{32}$P from [$\gamma$-$^{32}$P]ATP into histone. The degree of enzyme blockade at each test compound concentration is calculated from

$$\frac{\text{Cpm incorporated + test compound + enzyme}}{\text{Cpm incorporated - test compound + enzyme}} \times 100\%$$

The $IC_{50}$ value is that concentration of test compound which reduces by 50% the protein kinase-induced incorporation of the $^{32}$P under the assay conditions described above.

The results obtaining in the above test with representative compounds of formula I are given in the following Table.

Table

| Product of Example No. | $IC_{50}$ (nM) |
|---|---|
| 10 | 3.3 |
| 11 | 5.5 |

The compounds of formula I and their aforementioned salts can be used as medicaments, for example, in the form of pharmaceutical preparations. The pharmaceutical preparations can be administered orally, for example in the form of tablets, coated tablets, dragees, hard and soft gelatine capsules, solutions, emulsions or suspensions. However, they can also be administered rectally (e.g. in the form of suppositories) or parenterally (e.g. in the form of injection solutions).

For the manufacture of pharmaceutical preparations the compounds of formula I and their aforementioned salts can be formulated with therapeutically inert, inorganic or organic carriers. Lactose, maize starch or derivatives thereof, talc, stearic acid or its salts and the like can be used, for example, as such carriers for tablets, coated tablets, dragees and hard gelatine capsules. Suitable carriers for soft gelatine capsules are, for example, vegetable oils, waxes,

fats, semi-solid and liquid polyols and the like. Depending on the nature of the active substance no carriers are, however, generally required in the case of soft gelatine capsules. Suitable carriers for the manufacture of solutions and syrups are, for example, water, polyols, saccharose, invert sugar, glucose and the like. Suitable carriers for injection solutions are, for example, water, alcohols, polyols, glycerine, vegetable oils and the like. Suitable carriers for suppositories are, for example, natural or hardened oils, waxes, fats, semi-liquid polyols and the like.

The pharmaceutical preparations can also contain preserving agents, solubilizing agents, stabilizing agents, wetting agents, emulsifying agents, sweetening agents, colouring agents, flavouring agents, salts for varying the osmotic pressure, buffers, coating agents or antioxidants. They can also contain still other therapeutically valuable substances. Medicaments containing a compound of formula I or a salt thereof as defined above and a therapeutically inert carrier as well as a process for the manufacture of such medicaments are also objects of the present invention. This process comprises bringing a compound of formula I or a salt thereof as defined above into a galenical administration form together with a therapeutically inert carrier material and, if desired, one or more other therapeutically active substances.

As mentioned above, the compounds of formula I and their aforementioned salts can be used in the control or prevention of illnesses, especially in the control or prevention of inflammatory, immunological, bronchopulmonary, dermatological and cardiovascular disorders, for the treatment of asthma, AIDS or diabetic complications or for the stimulation of hair growth. The dosage can vary within wide limits and will, of course, be adjusted to the individual requirements in each particular case. In general, in the case of oral administration to adults, a daily dosage of about 5 mg to about 500 mg should be appropriate, although the upper limit may be exceeded when this is found to be expedient. The daily dosage can be administered as a single dose or in divided doses.

The following Examples illustrate the present invention:

Example 1

A solution of 1.25 g (2.32 mmol) of 3-[8(R or S)-[1(R or S)-tert-butoxyformamidoethyl]-6,7,8,9-tetrahydropyrido[1,2-a]indol-10-yl]-4-(1-methyl-3-indolyl)-1H-pyrrole-2,5-dione (diastereomer A) in 10 ml of ethyl acetate was treated with 30 ml of a saturated solution of hydrogen chloride in ethyl acetate and stirred at room temperature for 18 hours. The solid obtained was filtered off and dried to give 1.0 g of 3-[8(R or S)-[1(R or S)-aminoethyl]-6,7,8,9-tetrahydropyrido[1,2-a]indol-10-yl]-4-(1-methyl-3-indolyl)-1H-pyrrole-2,5-dione hydrochloride (diastereomer A) as a red solid of melting point 324-325°C.

The 3-[8(R or S)-[1(R or S)-tert-butoxyformamidoethyl]-6,7,8,9-tetrahydropyrido[1,2-a]indol-10-yl]-4-(1-methyl-3-indolyl)-1H-pyrrole-2,5-dione (diastereomer A) used as the starting material was prepared as follows:

(i) A stirred suspension of 27 g (126 mmol) of 6,7,8,9-tetrahydropyrido[1,2-a]indole-8-carboxylic acid in 30 ml of water and 450 ml of acetone was cooled to 0°C and treated in succession with 14.7 g (145 mmol) of triethylamine and 17.3 g (159 mmol) of ethyl chloroformate. After 0.5 hour 6.3 ml of .880 ammonia was added and stirring was continued for 1 hour. The solvent was removed under reduced pressure and the residue was triturated with aqueous ethanol. The product was filtered off and dried to give 13 g of 6,7,8,9-tetrahydropyrido[1,2-a]indole-8-carboxamide as a white solid of melting point 165-168°C.

(ii) 25.7 g (126 mmol) of trifluoroacetic anhydride were added dropwise to a stirred suspension of 26.5 g (123 mmol) of 6,7,8,9-tetrahydropyrido[1,2-a]indole-8-carboxamide and 23.4 g (300 mmol) of pyridine in 500 ml of dry dioxan at 10°C. After completion of the addition the solvent was removed under reduced pressure and the residue was crystallized from methanol to give 13 g of 6,7,8,9-tetrahydropyrido[1,2-a]indole-8(RS)-carbonitrile as a light tan solid of melting point 106-109°C.

(iii) A solution of 1.4 g (7.2 mmol) of 6,7,8,9-tetrahydropyrido[1,2-a]indole-8(RS)-carbonitrile in 400 ml of dry toluene was treated with 7 ml (21 mmol) of 3M solution of methylmagnesium chloride in tetrahydrofuran and the solution obtained was heated to reflux under nitrogen for 0.5 hour. The solution was then added to 17 ml (17 mmol) of a 1M solution of lithium aluminium hydride in tetrahydrofuran. The solution obtained was heated to reflux for 15 minutes, cooled and treated dropwise with about 20 ml of water. The precipitate was filtered off and washed with 100 ml of ethyl acetate and the combined filtrate and washings were evaporated under reduced pressure to give 1.55 g of a light brown oil.

The oil was dissolved in 70 ml of dry dichloromethane and treated with 1.5 g (15 mmol) of triethylamine followed by 1.8 g (7.4 mmol) of di-tert.butyl dicarbonate at 0°C under nitrogen. The stirred solution was allowed to warm to room temperature and stirring was continued for 1 hour. The solvent was removed under reduced pressure and the product was purified by flash chromatography on silica gel using diethyl ether/petroleum ether (1:2) for the elution to give 925 mg of tert.butyl [1(R or S)-(6,7,8,9-tetrahydropyrido[1,2-a]indol-8(R or S)-yl)ethyl]carbamate as

a mixture of diastereomers in the form of a white solid of melting point 114-117°C.

(iv) A stirred solution of 3.0 g (9.57 mmol) of tert.butyl[1(R or S)-(6,7,8,9-tetrahydropyrido[1,2-a]indol-8(R or S)-yl)ethyl]carbamate in 150 ml of dichloromethane was treated dropwise at 0°C with 1.28 g (10.3 mmol) of oxalyl chloride. After 5 minutes the solvent was removed under reduced pressure and the residue was dissolved in 150 ml of dichloromethane and treated with 2.94 g (11 mmol) of isopropyl 1-methyl-3-indoleacetimidate hydrochloride and 4.38 g (43 mmol) of triethylamine at 0°C under nitrogen. After warming to room temperature the solution was stirred for 24 hours, washed with water and dried over magnesium sulphate. The solvent was removed by evaporation and the residue was dissolved in 30 ml of pyridine. This stirred solution was cooled to ice bath temperature and treated dropwise with 1.5 ml (10.8 mmol) of trifluoroacetic anhydride. After 15 minutes the solvent was removed under reduced pressure and the residue was partitioned between 200 ml of ethyl acetate and 200 ml of 0.2M hydrochloric acid. The organic layer was washed with 50 ml of sodium bicarbonate solution, dried over magnesium sulphate and evaporated to dryness. The residue was purified by flash chromatography on silica gel using ethyl acetate/petroleum ether (1:2) for the elution to give 1.35 g of 3-[8(R or S)-[1(R or S)-tert.butoxyformamidoethyl]-6,7,8,9-tetrahydropyrido[1,2-a]indol-10-yl]-4-(1-methyl-3-indolyl)-1H-pyrrole-2,5-dione (diastereomer A) in the form of a red solid of melting point 255-7°C. Further elution gave 1.38 g of diastereomer B as a red solid of melting point 230-233°C.

Example 2

In an analogous manner to that described in the first paragraph of Example 1, from 1.38 g of 3-[8(R or S)-[1(R or S)-tert.butoxyformamidoethyl]-6,7,8,9-tetrahydropyrido[1,2-a]indol-10-yl]-4-(1-methyl-3-indolyl)-1H-pyrrole-2,5-dione (diastereomer B), prepared as described in Example 1(iv), there were obtained 930 mg of 3-[8(R or S)-[1(R or S)-aminoethyl]-6,7,8,9-tetrahydropyrido[1,2-a]indol-10-yl]-4-(1-methyl-3-indolyl)-1H-pyrrole-2,5-dione hydrochloride (diastereomer B) as a red solid of melting point 254-258°C.

Example 3

In a manner analogous to that described in the first paragraph of Example 1, from 180 mg of 3-[8(R or S)-[1(R or S)-tert.butoxyformamidopropyl]-6,7,8,9-tetrahydropyrido[1,2-a]indol-10-yl]-4-(1-methyl-3-indolyl)-1H-pyrrole-2,5-dione (diastereomer A) there were obtained 115 mg of 3-[8(R or S)-[1(R or S)-aminopropyl]-6,7,8,9-tetrahydropyrido[1,2-a]indol-10-yl]-4-(1-methyl-3-indolyl)-1H-pyrrole-2,5-dione hydrochloride (diastereomer A) as a red solid of melting point 321-323°C.

The 3-[8(R or S)-[1(R or S)-tert.butoxyformamidopropyl]-6,7,8,9-tetrahydropyrido[1,2-a]indol-10-yl]-4-(1-methyl-3-indolyl)-1H-pyrrole-2,5-dione (diastereomer A) used as the starting material was prepared as follows:

(i) In a manner analogous to that described in Example 1(iii), from 500 mg (2.5 mmol) of 6,7,8,9-tetrahydropyrido[1,2-a]indole-8(RS)-carbonitrile and 2.5 ml (5 mmol) of a 2M solution of ethylmagnesium bromide in tetrahydrofuran there were obtained 480 mg of tert.butyl [1(R or S)-(6,7,8,9-tetrahydropyrido[1,2-a]indol-8(R or S)-yl)propyl]carbamate as a mixture of diastereomers in the form of a white solid of melting point 153-156°C.

(ii) In a manner analogous to that described in Example 1(iv), from 440 mg (1.34 mmol) of tert.butyl [1(R or S)-(6,7,8,9-tetrahydropyrido[1,2-a]indol-8(R or S)-yl)propyl]carbamate there were obtained 180 mg of 3-[8(R or S)-[1(R or S)-tert.butoxyformamidopropyl]-6,7,8,9-tetrahydropyrido[1,2-a]indol-10-yl]-4-(1-methyl-3-indolyl)-1H-pyrrole-2,5-dione (diastereomer A) as a red gum. Further elution gave 130 mg of diastereomer B as a red gum.

Example 4

In a manner analogous to that described in the first paragraph of Example 1, from 130 mg of 3-[8(R or S)-1(R or S)-tert.butoxyformamidopropyl]-6,7,8,9-tetrahydropyrido[1,2-a]indol-10-yl]-4-(1-methyl-3-indolyl)-1H-pyrrole-2,5-dione [diastereomer B, prepared as described in Example 3(ii)], there were obtained 65 mg of 3-[8(R or S)-[1(R or S)-aminopropyl]-6,7,8,9-tetrahydropyrido[1,2-a]indol-10-yl]-4-(1-methyl-3-indolyl)-1H-pyrrole-2,5-dione hydrochloride (diastereomer B) as a red solid of melting point 245-249°C.

Example 5

In a manner analogous to that described in the first paragraph of Example 1, from 400 mg (0.7 mmol) of 3-[8-(R or S)-[1(R or S)-tert.butoxyformamidobutyl]-6,7,8,9-tetrahydropyrido[1,2-a]indol-10-yl]-4-(1-methyl-3-indolyl)-1H-

pyrrole-2,5-dione (diastereomer A) there were obtained 310 mg of 3-[8(R or S)-[1(R or S)-aminobutyl]-6,7,8,9-tetrahydropyrido[1,2-a]indol-10-yl]-4-(1-methyl-3-indolyl)-1H-pyrrole-2,5-dione hydrochloride (diastereomer A) as a red solid of melting point 237-241°C.

The 3-[8-(R or S)-[1(R or S)-tert.butoxyformamidobutyl]-6,7,8,9-tetrahydropyrido[1,2-a]indol-10-yl]-4-(1-methyl-3-indolyl)-1H-pyrrole-2,5-dione (diastereomer A) used as the starting material was prepared as follows:

In a manner analogous to that described in Example 1(iii) and (iv), from 1.0 g (5 mmol) of 6,7,8,9-tetrahydropyrido[1,2-a]indole-8(RS)-carbonitrile and 5.0 ml (10 mmol) of a 2M solution of n-propylmagnesium chloride in diethyl ether there were obtained 470 mg of 3-[8(R or S)-[1(R or S)-tert.butoxyformamidobutyl]-6,7,8,9-tetrahydropyrido[1,2-a]indol-10-yl]-4-(1-methyl-3-indolyl)- 1H-pyrrole-2,5-dione (diastereomer A) as a red solid of melting point 227-9°C. Further elution gave 285 mg of diastereomer B as a red solid of melting point 169-172°C.

Example 6

In a manner analogous to that described in the first paragraph of Example 1, from 270 mg (0.48 mmol) of 3-[8(R or S)-1(R or S)-tert.butoxyformamidobutyl]-6,7,8,9-tetrahydropyrido[1,2-a]indol-10-yl]-4-(1-methyl-3-indolyl)-1H-pyrrole-2,5-dione (diastereomer B), prepared as described in the second paragraph of Example 5, there were obtained 210 mg of 3-[8(R or S)-[1(R or S)-aminobutyl]-6,7,8,9-tetrahydropyrido[1,2-a]indol-10-yl]-4-(1-methyl-3-indolyl)-1H-pyrrole-2,5-dione hydrochloride (diastereomer B) as a red solid of melting point 235-238°C.

Example 7

In a manner analogous to that described in the first paragraph of Example 1, from 400 mg (0.7 mmol) of 3-[8(R or S)-[1(R or S)-tert.butoxyformamido-2-methylpropyl]-6,7,8,9-tetrahydropyrido[1,2-a]indol-10-yl]-4-(1-methyl-3-indolyl)-1H-pyrrole-2,5-dione (diastereomer A) there were obtained 300 mg of 3-[8(R or S)-[1(R or S)-amino-2-methylpropyl]-6,7,8,9-tetrahydropyrido[1,2-a]indol-10-yl]-4-(1-methyl-3-indolyl)-1H-pyrrole-2,5-dione hydrochloride (diastereomer A) as a red solid of melting point 254-256°C.

The 3-[8(R or S)-[1(R or S)-tert.butoxyformamido-2-methylpropyl]-6,7,8,9-tetrahydropyrido-[1,2-a]indol-10-yl]-4-(1-methyl-3-indolyl)-1H-pyrrole-2,5-dione (diastereomer A) used as the starting material was prepared as follows:

(i) A solution of 2.0 g (10 mmol) of 6,7,8,9-tetrahydropyrido[1,2-a]indole-8(RS)-carbonitrile in 150 ml of dry toluene was treated with 10 ml (20 mmol) of a 2M solution of isopropylmagnesium chloride in tetrahydrofuran and the solution obtained was heated to reflux for 20 minutes. The solution was cooled and treated dropwise with 24 ml (24 mmol) of a 1M solution of lithium aluminium hydride in tetrahydrofuran. The solution obtained was stirred at room temperature for 1.5 hours, cooled and treated dropwise with about 20 ml of water. The precipitate was filtered off, washed with 100 ml of dichloromethane and the combined filtrates were evaporated under reduced pressure to give 7.2 g of pale brown oil. The oil was dissolved in 100 ml of dry dichloromethane and treated with 2.34 g (23.4 mmol) of triethylamine followed by 2.8 g (11.6 mmol) of di-tert.-butyldicarbonate at 0°C under nitrogen. The stirred solution was allowed to warm to room temperature and stirring was continued for 70 hours. The solvent was removed under reduced pressure and the products were purified by flash chromatography on silica gel using diethyl ether/petroleum ether (1:2) for the elution to give 800 mg of tert.butyl [1(R or S)-[6,7,8,9-tetrahydropyrido[1,2-a]indol-8(R or S)-yl]-2-methylpropyl]carbamate (diastereomer A) as a white solid of melting point 118-9°C. Further elution gave 420 mg of diastereomer B as a white solid of melting point 128-129°C.

(ii) A stirred solution of 770 mg (2.25 mmol) of tert.butyl [1(R or S)-[6,7,8,9-tetrahydropyrido[1,2-a]indol-8(R or S)-yl]-2-methylpropyl]carbamate (diastereomer A) in 30 ml of dichloromethane at 0°C was treated dropwise with 287 mg (2.4 mmol) of oxalyl chloride. After 5 minutes the solvent was removed under reduced pressure and the residue was dissolved in 30 ml of dichloromethane and treated with 686 mg (2.57 mmol) of isopropyl 1-methyl-3-indoleacetimidate hydrochloride and 1.02 g (10 mmol) of triethylamine at 0°C under nitrogen. After warming to room temperature the solution was stirred for 18 hours, washed with 50 ml of water and dried over magnesium sulphate. The solvent was removed by evaporation and the residue was dissolved in 10 ml of pyridine. The stirred solution was cooled to ice bath temperature and treated dropwise with 664 µl (4.8 mmol) of trifluoroacetic anhydride. After 15 minutes 50 ml of ethyl acetate was added and the organic phase was washed with water and 2M hydrochloric acid, dried over magnesium sulphate and evaporated to dryness. The residue was purified by flash chromatography on silica gel using ethyl acetate/petroleum ether (1:2) for the elution to give 460 mg of 3-[8(R or S)-[1(R or S)-tert.butoxyformamido-2-methylpropyl]-6,7,8,9-tetrahydropyrido[1,2-a]indol-10-yl]-4-(1-methyl-3-indolyl)-1H- pyrrole-2,5-dione (diastereomer A) as a red solid of melting point 223-224°C.

Example 8

In an analogous manner to that described in the first paragraph of Example 1, from 270 mg (0.48 mmol) of 3-[8(R or S)-[1(R or S)-tert.butoxyformamido-2-methylpropyl]-6,7,8,9-tetrahydropyrido[1,2-a]indol-10-yl]-4-(1-methyl-3-indolyl)-1H-pyrrole-2,5-dione (diastereomer B) there were obtained 180 mg of 3-[8(R or S)-[1(R or S)-amino-2-methylpropyl]-6,7,8,9-tetrahydropyrido[1,2-a]indol-10-yl]-4-9-(1-methyl-3-indolyl)-1H-pyrrole-2,5-dione hydrochloride (diastereomer B) as a red solid of melting point 248-250°C.

The 3-[8(R or S)-[1(R or S)-tert.butoxyformamido-2-methylpropyl]-6,7,8,9-tetrahydropyrido[1,2-a]indol-10-yl]-4-(1-methyl-3-indolyl)-1H-pyrrole-2,5-dione (diastereomer B) used as the starting materal was prepared as follows:

In an analogous manner to that described in Example 7(ii), from 385 mg of tert.butyl [1(R or S)-[6,7,8,9-tetrahydropyrido[1,2-a]indol-8(R or S)-yl]-2-methylpropyl]carbamate (diastereomer B), prepared as described in Example 7(i), there were obtained 270 mg of 3-[8(R or S)-[1(R or S)-tert.butoxyformamido-2-methylpropyl]-6,7,8,9-tetrahydropyrido[1,2-a]indol-10-yl]-4-(1-methyl-3-indolyl)-1H-pyrrole-2,5-dione (diastereomer B) as a red gum.

Example 9

In a manner analogous to that described in the first paragraph of Example 1, from 1.2 g (2.17 mmol) of 3-[8(S)-[1(R or S)-tert.butoxyformamidopropyl]-6,7,8,9-tetrahydropyrido[1,2-a]indol-10-yl]-4-(1-methyl-3-indolyl)-1H-pyrrole-2,5-dione (diastereomer A) there were obtained 850 mg of 3-[8(S)-[1(R or S)-aminopropyl]-6,7,8,9-tetrahydropyrido[1,2-a]indol-10-yl]-4-(1-methyl-3-indolyl)-1H-pyrrole-2,5-dione hydrochloride (diastereomer A) as a red solid of melting point 304-308°C.

The 3-[8(S)-[1(R or S)-tert.butoxyformamidopropyl]-6,7,8,9-tetrahydropyrido[1,2-a]indol-10-yl]-4-(1-methyl-3-indolyl)-1H-pyrrole-2,5-dione (diastereomer A) used as the starting material was prepared as follows:

(i) An ice-cooled solution of 50.0 g (248 mmol) of 8(S)-hydroxymethyl-6,7,8,9-tetrahydropyrido[1,2-a]indole in 500 ml of dichloromethane was treated with 50 ml (358 mmol) of triethylamine and 52.0 g (298 mmol) of methanesulphonic anhydride over a period of 20 minutes. After 2 hours 250 ml of water were added and the organic phase was washed in succession with two 250 ml portions of saturated sodium bicarbonate solution and 200 ml of water. The organic phase was then dried over magnesium sulphate and evaporated. The residual solid was triturated with ether, filtered off and dried in vacuo to give 65.4g of [6,7,8,9-tetrahydropyrido[1,2-a]indol-8(S)-yl]methyl methanesulphonate as a pale pink solid of melting point 114-5°C; $[\alpha]^{20}_{D}$ = -39.7° (c = 1%, $CH_2Cl_2$).

(ii) 18.0 g (367 mmol) of sodium cyanide were added to a solution of 65.0 g (233 mmol) of (6,7,8,9-tetrahydropyrido[1,2-a]indol-8(S)-yl)methyl methanesulphonate in 500 ml of dimethylformamide and the mixture was heated at 70°C for 24 hours. The mixture was partitioned between 1000 ml of water and 600 ml of ethyl acetate. The aqueous phase was extracted twice with 700 ml of ethyl acetate each time and the combined extracts were washed in twice with 500 ml of water each time, dried over magnesium sulphate and evaporated. The brown solid obtained was dissolved in ethyl acetate and the solution was filtered through a pad of silica gel. The solvent was removed in a vacuum and the residue was crystallized from methanol to give 25.8 g of 6,7,8,9-tetrahydropyrido[1,2-a]indole-8(S)-acetonitrile as a light brown solid of melting point 100-101°C; $[\alpha]^{20}_{D}$ -40.6° (c = 0.84%, $CH_2Cl_2$).

(iii) A solution of 27.0 g (129 mmol) of 6,7,8,9-tetrahydropyrido[1,2-a]indole-8(S)-acetonitrile and 120 ml of 2M sodium hydroxide in 400 ml of 1,2-ethanediol was heated to reflux for 4 hours. 400 ml of ethyl acetate were added and the organic phase was washed in succession with 500 ml of water, 150 ml of 2M hydrochloric acid and three 500 ml portions of water, dried over magnesium sulphate and evaporated to give 29 g of [8(S)-(6,7,8,9-tetrahydropyrido[1,2-a]indolyl]acetic acid as a pale pink solid of melting point 118-120°C.

(iv) A solution of 29 g (127 mmol) of 8(S)-(6,7,8,9-tetrahydropyrido[1,2-a]indolyl)acetic acid and 5 ml of concentrated sulphuric acid in 500 ml of methanol was heated to reflux for 1 hour. The mixture was concentrated under reduced pressure and the product was filtered off and dried to give 28.4 g of methyl [8(S)-(6,7,8,9-tetrahydropyrido[1,2-a]indolyl]acetate as a pale pink solid of melting point 84-87°C.

(v) 10 g (100 mmol) of diisopropylamine in 60 ml of dry tetrahydrofuran were treated at 0°C under nitrogen with 63 ml (100 mmol) of a 1.6M solution of n-butyllithium in hexane and stirred for 15 minutes. The solution obtained was cooled to -78°C and a solution of 14.9 g (61.3 mmol) of methyl [8(S)-6,7,8,9-tetrahydropyrido[1,2-a]indolyl]acetate in 60 ml of tetrahydrofuran was added dropwise. After 30 minutes 15.6 g (100 mmol) of ethyl iodide were added and the mixture was stirred for 0.5 hour. A further 8 g of ethyl iodide were then added and the mixture was allowed to warm to room temperature for about 1 hour. The mixture was partitioned between 300 ml of diethyl ether and 20

ml of 2M hydrochloric acid. The organic phase was washed three times with 300 ml of water each time, dried over magnesium sulphate and evaporated to give 17 g of a pale orange oil. A sample was crystallized from methanol to give methyl alpha (R or S)-ethyl-6,7,8,9-tetrahydropyrido[1,2-a]indole-8(S)-acetate (diastereomers A + B) as a pale pink solid of melting point 87-90°C.

(vi) A solution of 17 g (63 mmol) of methyl alpha (R or S)-ethyl-6,7,8,9-tetrahydropyrido[1,2-a]indole-8(S)-acetate (diastereomers A + B) in 150 ml of methanol was treated with 130 ml of 2M sodium hydroxide and heated to reflux for 18 hours. The cooled solution was added to 150 ml of 4M hydrochloric acid and the resulting precipitate was filtered off and partitioned between 200 ml of dichloromethane and 100 ml of water. The organic phase was separated, dried over magnesium sulphate and evaporated to give 15 g of alpha (R or S)-6,7,8,9-tetrahydropyrido[1,2-a]indole-8(S)-acetic acid (diastereomers A + B) as a pale pink solid.

(vii) A stirred solution of 15 g (58.4 mmol) of alpha (R or S)-6,7,8,9-tetrahydropyrido[1,2-a]indole-8(S)-acetic acid (diastereomers A + B) in 450 ml of toluene was treated with 6.5 g (65 mmol) of triethylamine and 18.6 g (67.5 mmol) of diphenylphosphonyl azide at room temperature under nitrogen. After 1 hour at room temperature the mixture was heated to reflux for 0.5 hour, cooled and the solvent was removed under reduced pressure. The residue was purified by flash chromatography on silica gel using diethyl ether/petroleum ether (1:3) for the elution to give 14 g of the isocyanate as a colourless oil. The oil was dissolved in 400 ml of dioxan and 150 ml of 2M hydrochloric acid and the solution obtained was stirred at room temperature for 18 hours. The solution was concentrated and partitioned between 300 ml of ethyl acetate and 2M sodium hydroxide solution. The aqueous layer was extracted with 100 ml of ethyl acetate and the combined organic solutions were washed twice with 400 ml of water each time, dried over magnesium sulphate and evaporated to dryness to give 7.7 g of a cream coloured foam.

This foam in 200 ml of dry dichloromethane was treated with 7.6 g (75 mmol) of triethylamine and 10.9 g (50 mmol) of di-tert.butyl dicarbonate and the mixture was stirred at room temperature for 3 hours. The solvent was removed under reduced pressure and the residue was subjected to flash chromatography on silica gel using diethyl ether/petroleum ether (1:2) for the elution to give 4.5 g of tert.butyl [1(R or S)-[6,7,8,9-tetrahydropyrido[1,2-a]indole-8(S)-yl]propyl]carbamate (diasteroisomers A + B) as a white solid of melting point 152-158°C.

(viii) In a manner analogous to that described in Example 1(iv), from 4.3 g (13.1 mmol) of tert.butyl [1(R or S)-[6,7,8,9-tetrahydropyrido[1,2-a]indol-8(S)-yl]propyl]carbamate (diastereomers A + B) there were obtained 1.2 g of 3-[8(S)-[1(R or S)-tert.butoxyformamidopropyl]-6,7,8,9-tetrahydropyrido[1,2-a]indol-10-yl]-4-(1-methyl-3- indolyl)-1H-pyrrole-2,5-dione (diastereomer A) and 2.0 g of diastereomer B, both as red solids.

Example 10

In a manner analogous to that described in the first paragraph of Example 1, from 2.0 g (3.62 mmol) of 3-[8(S)-[1(R or S)-tert.butoxyformamidopropyl]-6,7,8,9-tetrahydropyrido[1,2-a]indol-10-yl]-4-(1-methyl-3-indolyl)-1H-pyrrole-2,5-dione (diastereomer B), prepared as described in Example 9, there were obtained 1.28 g of 3-[8(S)-[1(R or S)-aminopropyl]-6,7,8,9-tetrahydropyrido[1,2-a]indol-10-yl]-4-(1-methyl-3-indolyl)-1H-pyrrole-2,5-dione hydrochloride (diastereomer B) as a red solid of melting point 247-253°C.

Example 11

In a manner analogous to that described in the first paragraph of Example 1, from 1.46 g of 3-[8(S)-[1(S)-tert.butoxyformamido-2-methylpropyl]-6,7,8,9-tetrahydropyrido[1,2-a]indol-10-yl]-4-(1-methyl-3-indolyl)-1H-pyrrole-2,5-dione there were obtained 1.29 g of 3-[8(S)-[1(S)-amino-2-methylpropyl]-6,7,8,9-tetrahydropyrido[1,2-a]indol-10-yl-4-(1-methyl-3-indolyl)-1H-pyrrole-2,5-dione hydrochloride as a red solid of melting point 253-256°C.

The 3-[8(S)-[1(S)-tert.butoxyformamido-2-methylpropyl]-6,7,8,9-tetrahydropyrido[1,2-a]indol-10-yl]-4-(1-methyl-3-indolyl)-1H-pyrrole-2,5-dione used as the starting material was prepared as follows:

(i) To 40 g (114 mmol) of 6,7,8,9-tetrahydropyrido[1,2-a]indole-8(S)-carboxylic acid 1-menthyl ester were added 50 ml of concentrated sulphuric acid and the mixture obtained was stirred until all starting material had dissolved (about 20 minutes). The solution was poured carefully into 1500 ml of ice-water and the resulting precipitate was filtered off, washed with petroleum ether/toluene (3:1) and dried to give 24.2 g of 6,7,8,9-tetrahydropyrido[1,2-a]indole-8(S)-carboxylic acid as a white solid of melting point 251-253°C.

(ii) A stirred suspension of 24.0 g (111 mmol) of 6,7,8,9-tetrahydropyrido[1,2-a]indole-8(S)-carboxylic acid in 500 ml of dichloromethane was treated in succession at 0°C with 24 ml (138 mmol) of diisopropylethylamine, 13.24 g

(136 mmol) of N,O-dimethylhydroxylamine hydrochloride, 10 mg of dimethylaminopyridine and 23.04 g (112 mmol) of dicyclohexylcarbodiimide. The mixture obtained was stirred at room temperature for 18 hours and filtered, and the solid was washed twice with 100 ml of dichloromethane each time. The combined filtrates were evaporated to dryness and the residue was purified by flash chromatography on silica gel using ethyl acetate/petroleum ether (1:3) for the elution to give 22.6 g of a white solid. A sample was triturated with ether/petroleum ether to give 6,7,8,9-tetrahydro-N-methoxy-N-methyl-pyrido[1,2-a]indole-8(S)-carboxamide as a white solid of melting point 78-80°C.

(iii) A stirred solution of 10.0 g (38.7 mmol) of 6,7,8,9-tetrahydro-N-methoxy-N-methyl-pyrido[1,2-a]indole-8(S)-carboxamide in 250 ml of tetrahydrofuran was treated dropwise at 0°C with 60 ml (120 mmol) of a 2M solution of isopropylmagnesium chloride in tetrahydrofuran. The mixture was stirred at room temperature for 18 hours and poured into 250 ml of saturated ammonium chloride solution. The aqueous phase was washed four times with 100 ml of diethyl ether each time and the combined ethereal extracts were washed with 200 ml of brine, dried over magnesium sulphate and evaporated to dryness. The product was purified by flash chromatography on silica gel using ethyl acetate/petroleum ether (1:3) for the elution to give 4.4 g of isopropyl 6,7,8,9-tetrahydropyrido[1,2-a]indol-8(S)-yl ketone as a white solid of melting point 78-79°C.

(iv) A suspension of 4.0 g (16.6 mmol) of isopropyl 6,7,8,9-tetrahydropyrido[1,2-a]indol-8(S)-yl ketone in 120 ml of ethanol was treated with a solution of 2.30 g (33 mmol) of hydroxylamine hydrochloride and 1.0 g (25 mmol) of sodium hydroxide in 20 ml of water. The mixture obtained was heated to reflux for 3.5 hour, cooled and filtered. The solid obtained was dried to give 3.54 g of oxime as a white solid.

The oxime was dissolved in 150 ml of dry tetrahydrofuran and treated with 12.5 ml (12.5 mmol) of a 1M solution of lithium aluminium hydride in tetrahydrofuran. The solution obtained was heated to reflux for 3 hours under nitrogen, cooled and cautiously treated with 150 ml of water. The mixture was extracted with 200 ml of ethyl acetate and then with two 150 ml portions of ethyl acetate, and the combined organic extracts were dried over magnesium sulphate and evaporated to dryness. The residue was dissolved in 150 ml of dichloromethane and the solution obtained was treated with 3 ml (21.5 mmol) of triethylamine and 3.4 g (15.6 mmol) of di-tert.butyl dicarbonate and stirred for 18 hours. The mixture was washed with 150 ml of saturated ammonium chloride solution, dried over magnesium sulphate and evaporated under reduced pressure. Purification by flash chromatography on silica gel using diethyl ether/petroleum ether (1:3) for the elution gave 1.4 g of tert.butyl [1(R)-(6,7,8,9-tetrahydropyrido[1,2-a]indol-8(S)-yl)-2-methylpropyl]carbamate as a white solid of melting point 122-124°C. Further elution gave 1.1g of tert.butyl [1(S)-[6,7,8,9-tetrahydropyrido[1,2-a]indol-8(S)-yl]-2-methylpropyl]carbamate as a white solid of melting point 154-155°C.

(v) In a manner analogous to that described in Example 1(iv), from 1.1 g of tert.butyl [1(S)-[6,7,8,9-tetrahydropyrido[1,2-a]indol-8(S)-yl]-2-methylpropyl]carbamate there were obtained 1.46 g of 3-[8(S)-[1(S)-tert.butoxyformamido-2-methylpropyl]-6,7,8,9-tetrahydropyrido[1,2-a]indol-10-yl]-4-(1-methyl-3-indolyl)-1H-pyrrole-2,5-dione as a red foam.

Example 12

In a manner analogous to that described in the first paragraph of Example 1, from 0.5 g (0.88 mmol) of 3-[8(S)-[1(R)-tert.butoxyformamido-2-methylpropyl]-6,7,8,9-tetrahydropyrido[1,2-a]indol-10-yl]-4-(1-methyl-3-indolyl)-1H-pyrrole-2,5-dione there was obtained 0.41 g of 3-[8(S)-[1(R)-amino-2-methylpropyl]-6,7,8,9-tetrahydropyrido[1,2-a]indol-10-yl]-4-(1-methyl-3-indolyl)-1H-pyrrole-2,5-dione hydrochloride as a red solid of melting poing 235-242.

The 3-[8(S)-[1(R)-tert.butoxyformamido-2-methylpropyl]-6,7,8,9-tetrahydropyrido[1,2-a]indol-10-yl]-4-(1-methyl-3-indolyl)-1H-pyrrole-2,5-dione used as the starting material was prepared as follows:

In a manner analogous to that described in Example 1(iv), from 0.94 g of tert.butyl [1(R)-[6,7,8,9-tetrahydropyrido[1,2-a]indol-8(S)-yl]-2-methylpropyl]carbamate, prepared as described in Example 11(i) - (iv), there were obtained 1.05 g of 3-[8(S)-[1(R)-tert.butoxyformamido-2-methylpropyl]-6,7,8,9-tetrahydropyrido[1,2-a]indol-10-yl]-4-(1-methyl-3-indolyl)-1H-pyrrole-2,5-dione as a red foam.

Example 13

In a manner analogous to that described in the first paragraph of Example 1, from 100 mg of 3-[8(R or S)-[alpha (R or S)-tert.butoxyformamidobenzyl]-6,7,8,9-tetrahydropyrido[1,2-a]-indol-10-yl]-4-(1-methyl-3-indolyl)-1H-pyrrole-2,5-dione (diastereomer A) there were obtained 50 mg of 3-[8(R or S) [alpha (R or S)-aminobenzyl]-6,7,8,9-tetrahydropyrido[1,2-a]indol-10-yl]-4-(1-methyl-3-indolyl)-1H-pyrrole-2,5-dione hydrochloride (diastereomer A) as a red solid of

melting point 234-237°C.

The 3-[8(R or S) [alpha(R or S)-tert.butoxyformamidobenzyl]-6,7,8,9-tetrahydropyrido[1,2-a]indol-10-yl]-4-(1-methyl-3-indolyl)-1H-pyrrole-2,5-dione (diastereomer A) used as the starting material was prepared as follows:

(i) In a manner analogous to that described in Example 1(iii) from 1.0 g (5.1 mmol) of 6,7,8,9-tetrahydropyrido[1,2-a]indole-8(RS)-carbonitrile and 3.7 ml (11 mmol) of a 3M solution of phenylmagnesium bromide in tetrahydrofuran there was obtained 0.9 g of tert.butyl [alpha (R or S)-(6,7,8,9-tetrahydropyrido[1,2-a]indol-8(R or S)-yl)benzyl]carbamate as a mixture of diastereomers in the form of a white solid of melting point 160-165°C.

(ii) In a manner analogous to that described in Example 1(iv), from 800 mg (2.1 mmol) of tert.butyl [alpha (R or S)-(6,7,8,9-tetrahydropyrido[1,2-a]indol-8-(R or S)-yl)benzyl]carbamate there were obtained 330 mg of 3-[8(R or S)-[alpha (R or S)-tert.butoxyformamidobenzyl]-6,7,8,9-tetrahydropyrido[1,2-a]-indol-10-yl]-4-(1-methyl-3-indolyl)-1H-pyrrole-2,5-dione (diastereomer A) as a red gum. Further elution gave 280 mg of diastereomer B as a red gum.

Example 14

In a manner analogous to that described in the first paragraph of Example 1, from 200 mg of 3-[8(R or S)-[alpha (R or S)-tert.butoxyformamidobenzyl]-6,7,8,9-tetrahydropyrido[1,2-a]indol-10-yl]-4-(1-methyl-3-indolyl)-1H-pyrrole-2,5-dione (diastereomer B), prepared as described in Example 13(ii), there were obtained 70 mg of 3-[8(R or S)-[alpha (R or S)-aminobenzyl]-6,7,8,9-tetrahydropyrido[1,2-a]-indol-10-yl]-4-(1-methyl-3-indolyl)-1H-pyrrole-2,5-dione hydrochloride (diastereomer B) as a red solid of melting point 226-233°C.

Example 15

In a manner analogous to that described in the first paragraph of Example 1, from 200 mg of 3-[8(S)-[(R or S)-(tert.butoxyformamido)(cyclopentyl)methyl]-6,7,8,9-tetrahydropyrido[1,2-a]indol-10-yl]-4-(1-methyl-3-indolyl)-1H-pyrrole-2,5-dione (diastereomer A) there were obtained 150 mg of 3-[8(S)-[(R or S)-(amino)(cyclopentyl)methyl]-6,7,8,9-tetrahydropyrido[1,2-a]indol-10-yl]-4-(1-methyl-3-indolyl)-1H-pyrrole-2,5-dione hydrochloride (diastereomer A) as a red solid of melting point 236-241°C.

The 3-[8(S)-[(R or S)-(tert.butoxyformamido)(cyclopentyl)methyl]-6,7,8,9-tetrahydropyrido[1,2-a]indol-10-yl]-4-(1-methyl-3-indolyl)-1H-pyrrole-2,5-dione used as the starting material was prepared as follows:

(i) In a manner analogous to that described in Example 11(iii), from 2.0 g (7.75 mmol) of 6,7,8,9-tetrahydro-N-methoxy-N-methylpyrido[1,2-a]indole-8(S)-carboxamide and 15 ml (30 mmol) of a 2M solution of cyclopentylmagnesium chloride in diethyl ether there were obtained 1.1 g of cyclopentyl 6,7,8,9-tetrahydropyrido]1,2-a]indol-8(S)-yl ketone as a pale yellow solid of melting point 69°C.

(ii) In a manner analogous to that described in Example 11(iv), from 1.05 g (3.9 mmol) of cyclopentyl 6,7,8,9-tetrahydropyrido[1,2-a]indol-8(S)-yl ketone there were obtained 330 mg of 8(S)-[(R or S)-(tert.butoxyformamido)(cyclopentyl)methyl]-6,7,8,9-tetrahydropyrido[1,2-a]indole (diastereomer A) as a white solid of melting point 140-143°C. Further elution gave 430 mg of diastereomer B as a white solid of melting point 58-63°C.

(iii) In a manner analogous to that described in Example 1(iv), from 300 mg of 8(S)-[(R or S)-tert.butoxyformamido)(cylopentyl)methyl]-6,7,8,9-tetrahydropyrido[1,2-a]indole (diastereomer A) there were obtained 200 mg of 3-[8(S)-[(R or S)-(tert.butoxyformamido)(cyclopentyl)methyl]-6,7,8,9-tetrahydropyrido[1,2-a]indol-10-yl]-4-(1-methyl-3-indolyl)-1H-pyrrole-2,5-dione as a red gum.

Example 16

In a manner analogous to that described in the first paragraph of Example 1, from 250 mg of 3-[8(S)-[(R or S)-(tert.butoxyformamido)(cyclopentyl)methyl]-6,7,8,9-tetrahydropyrido[1,2-a]indol-10-yl]-4-(1-methyl-3-indolyl)-1H-pyrrole-2,5-dione (diastereomer B) there were obtained 160 mg of 3-[8(S)-[(R or S)-(amino)(cyclopentyl)methyl]-6,7,8,9-tetrahydropyrido{1,2-a]indol-10-yl]-4-(1-methyl-3-indolyl)-1H-pyrrole-2,5-dione hydrochloride (diastereomer B) as a red solid of melting point 241-245°C.

The 3-[8(S)-[(R or S)-(tert.butoxyformamido)(cyclopentyl)methyl]-6,7,8,9-tetrahydropyrido[1,2-a]indol-10-yl]-4-(1-methyl-3-indolyl)-1H-pyrrole-2,5-dione used as the starting material was prepared as follows:

In a manner analogous to that described in Example 1(iv), from 400 mg of 8(S)-[(R or S)-(tert.butoxyformamido)(cyclopentyl)methyl]-6,7,8,9-tetrahydropyrido[1,2-a]indole (diastereomer B), prepared as described in Example

15(i) - (ii), there were obtained 250 mg of 3-[8(S)-[(R or S)-(tert.butoxyformamido)(cyclopentyl)methyl]-6,7,8,9-tetrahydropyrido[1,2-a]indol-10-yl]-4-(1-methyl-3-indolyl)-1H-pyrrole-2,5-dione as a red gum.

Example 17

In a manner analogous to that described in the first paragraph of Example 1, from 40 mg of 3-[2(R or S)-[1(R or S)-tert.butoxyformamido-2-methylpropyl]-2,3-dihydro-1H-pyrrolo[1,2-a]indol-9-yl]-4-(1-methyl-3-indolyl)-1H-pyrrole-2,5-dione (diastereomer A) there were obtained 20 mg of 3-[2-(R or S)-[1(R or S)-amino-2-methylpropyl]-2,3-dihydro-1H-pyrrolo[1,2-a]indol-9-yl]-4-(1-methyl-3-indolyl)-1H-pyrrole-2,5-dione hydrochloride (diastereomer A) as a red solid of melting point 224-230°C.

The 3-[2(R or S)-[1(R or S)-tert.butoxyformamido-2-methyl-propyl]-2,3-dihydro-1H-pyrrolo[1,2-a]indol-9-yl]-4-(1-methyl-3-indolyl)-1H-pyrrole-2,5-dione used as the starting material was prepared as follows:

(i) A solution of 8.0 g (35 mmol) of ethyl 2,3-dihydro-1H-pyrrolo[1,2-a]indole-2(RS)-carboxylate in 100 ml of ethanol and 100 ml of water was treated with 3.0 g (75 mmol) of sodium hydroxide. The mixture was heated at reflux for 15 minutes, then cooled and acidified with 60 ml (120 mmol) of 2M hydrochloric acid. The suspension was filtered and the solid was washed with 50 ml of water and then dried to give 5.9 g of 2,3-dihydro-1H-pyrrolo[1,2-a]indole-2(RS)-carboxylic acid as a white solid of melting point 171-173°C.

(ii) In a manner analogous to that described in Example 11(ii), from 4.0 g (20 mmol) of 2,3-dihydro-1H-pyrrolo[1,2-a]indole-2(RS)-carboxylic acid there were obtained 2.35 g of 2,3-dihydro-N-methoxy-N-methyl-1H-pyrrolo[1,2-a]indole-2(RS)-carboxamide as a white solid of melting point 87-88°C.

(iii) A suspension of 840 mg (35 mg atom) of magnesium turnings in 60 ml of tetrahydrofuran was treated dropwise with a solution of 4.4 g (37 mmol) of 2-bromopropene in 10 ml of tetrahydrofuran. The mixture was heated at reflux for a further 30 minutes, then cooled to 0°C and added at this temperature to a solution of 2.3 g (9.4 mmol) of 2,3-dihydro-N-methoxy-N-methyl-1H-pyrrolo[1,2-a]indole-2(RS)-carboxamide in 50 ml of tetrahydrofuran. After stirring at 0°C for 30 minutes the mixture was poured into 200 ml of saturated aqueous ammonium chloride solution. The solution was extracted with 200 ml of ethyl acetate and the organic phase was dried over magnesium sulphate and filtered. Addition of petroleum ether (b.p. 40-60°C) gave a precipitate which was filtered off and dried to give 1.6 g of a white solid. The solid was dissolved in 100 ml of ethanol and hydrogenated over 200 mg of 10% palladium-on-carbon at atmospheric pressure for 1 hour. The catalyst was removed by filtration and the filtrate was concentrated under reduced pressure until crystallization commenced. The product was filtered off and dried to give 1.55 g of isopropyl 2,3-dihydro-1H-pyrrolo[1,2-a]indol-2(RS)-yl ketone as a white solid of melting point 104-105°C.

(iv) In a manner analogous to that described in Example 11(iv), from 1.5 g of isopropyl 2,3-dihydro-1H-pyrrolo[1,2-a]indol-2(RS)-yl ketone there were obtained 830 mg of tert.butyl [1(R or S)-[2,3-dihydro-1H-pyrrolo-[1,2-a]indol-2(R or S)-yl]-2-methylpropyl]carbamate as a mixture of diastereomers. The mixture was stirred in 20 ml of ethyl acetate saturated with hydrogen chloride for 2 hours. The solid obtained was filtered off and purified by flash chromatography on silica gel using methanol/dichloromethane (1:10) for the elution to give 150 mg of 2(R or S)-[1(R or S)-amino-2-methylpropyl]-2,3-dihydro-1H-pyrrolo[1,2-a]indole hydrochloride (diastereomer A) in the form of a white solid. Further elution gave 150 mg of diastereomer B as a white solid.

(v) A solution of 100 mg (0.38 mmol) of 2(R or S)-[1(R or S)-amino-2-methylpropyl]-2,3-dihydro-1H-pyrrolo[1,2-a]indole hydrochloride (diastereomer A) in 30 ml of dichloromethane was treated with 110 mg (0.5 mmol) of di-tert.butyldicarbonate and 100 mg (1 mmol) of triethylamine and stirred for 72 hours. The solution was washed in succession with 30 ml of 1M hydrochloric acid and 30 ml of saturated aqueous sodium bicarbonate and then dried over magnesium sulphate. Filtration, concentration of the filtrate under reduced pressure and purification of the residue by flash chromatography using diethyl ether/petroleum ether (b.p. 40-60°C) (1:2) for the elution gave 100 mg of tert.butyl [1(R or S)-[2,3-dihydro-1H-pyrrolo[1,2-a]indol-2-(R or S)-yl]-2-methylpropyl]carbamate (diastereomer A) in the form of an oil.

(vi) In a manner analogous to that described in the first paragraph of Example 1, from 55 mg of tert.butyl [1(R or S)-[2,3-dihydro-1H-pyrrolo[1,2-a]indol-2-(R or S)-yl]-2-methylpropyl]carbamate (diastereomer A) there were obtained 40 mg of 3-[2(R or S)-[1(R or S)-tert.butoxyformamido-2-methylpropyl]-2,3-dihydro-1H-pyrrolo[1,2-a]indol-9-yl]-4-(1-methyl-3-indolyl)-1H-pyrrole-2,5-dione as a red oil

Example 18

In a manner analogous to that described in the first paragraph of Example 1, from 80 mg of 3-[2(R or S)-[1(R or S)-tert.butoxyformamido-2-methylpropyl]-2,3-dihydro-1H-pyrrolo[1,2-a]indol-9-yl]-4-(1-methyl-3-indolyl)-1H-pyrrole-2,5-dione (diastereomer B) there were obtained 40 mg of 3-[2(R or S)-amino-2-methylpropyl]2,3-dihydro-1H-pyrrolo[1,2-a]indol-9-yl]-4-(1-methyl-3-indolyl)-1H-pyrrole-2,5-dione hydrochloride (diastereomer B) as a red solid of melting point 220-225°C.

The 3-[2(R or S)-[1(R or S)-tert.butoxyformamido-2-methylpropyl]-2,3-dihydro-1H-pyrrolo-[1,2-a]indol-9-yl]-4-(1-methyl-3-indolyl)-1H-pyrrole-2,5-dione used as the starting material was prepared as follows:

(i) In a manner analogous to that described in Example 17(v), from 90 mg (0.34 mmol) of 2(R or S)-[1-(R or S)-amino-2-methylpropyl]-2,3-dihydro-1H-pyrrolo[1,2-a]indole hydrochloride (diastereomer B), prepared as described in Example 17(iv), there were obtained 100 mg of tert.butyl [1(R or S)-[2,3-dihydro-1H-pyrrolo[1,2-a]indol-2-(R or S)-yl]-2-methylpropyl]carbamate (diastereomer B) as an oil.

(ii) In a manner analogous to that described in Example 1(iv), from 100 mg of tert.butyl [1(R or S)-[2,3-dihydro-1H-pyrrolo[1,2-a]indol-2-(R or S)-yl]-2-methylpropyl]carbamate there were obtained 80 mg of 3-[2(R or S)-[1(R or S)-tert.butoxyformamido-2-methylpropyl]-2,3-dihydro-1H-pyrrolo[1,2-a]indol-9-yl]-4-(1-methyl-3-indolyl)-1H-pyrrole-2,5-dione as a red oil.

Example 19

In a manner analogous to that described in the first paragraph of Example 1, from 320 mg of 3-[8(RS)-[1(RS)-tert.butoxyformamido-2-methylpropyl]-7,8,9,10-tetrahydro-6H-azepino[1,2-a]indol-11-yl]-4-(1-methyl-3-indolyl)-1H-pyrrole-2,5-dione there were obtained 220 mg of 3-[8(RS)-[1(RS)-amino-2-methylpropyl]-7,8,9,10-tetrahydro-6H-azepino[1,2-a]indol-11-yl]-4-(1-methyl-3-indolyl)-1H-pyrrole-2,5-dione hydrochloride as a red solid of melting point 248-256°C.

The 3-[8(RS)-[1(RS)-tert.butoxyformamido-2-methylpropyl]7,8,9,10-tetrahydro-6H-azepino[1,2-a]indol-11-yl]-4-(1-methyl-3-indolyl)-1H-pyrrole-2,5-dione used as the starting material was prepared as follows:

(i) In a manner analogous to that described in Example 11(ii), from 1.0 g (4.4 mmol) of 7,8,9,10-tetrahydro-6H-azepino[1,2-a]indole-8(RS)-carboxylic acid there was obtained 0.8 g of 7,8,9,10-tetrahydro-N-methoxy-N-methyl-6H-azepino-[1,2-a]indole-8(RS)-carboxamide as a white solid of melting point 134-135°C.

(ii) In a manner analogous to that described in Example 17(iii), from 0.8 g (2.9 mmol) of 7,8,9,10-tetrahydro-N-methoxy-N-methyl-6H-azepino[1,2-a]indole-8(RS)-carboxamide there was obtained 0.56 g of isopropyl 7,8,9,10-tetrahydro-6H-azepino[1,2-a]indol-8(RS)-yl ketone as a white solid of melting point 79-80°C.

(iii) In a manner analogous to that described in Example 11(iv), from 0.56 g (2.2 mmol) of isopropyl 7,8,9,10-tetrahydro-6H-azepino[1,2-a]indol-8(RS)-yl ketone there were obtained 330 mg of tert.butyl [1(RS)-[7,8,9,10-tetrahydro-6H-azepino[1,2-a]indol-8(RS)-yl]-2-methylpropyl]carbamate as a mixture of diastereomers in the form of a white solid of melting point 152-153°C.

(iv) In a manner analogous to that described in Example 1(iv), from 300 mg tert.butyl [1(RS)-[7,8,9,10-tetrahydro-6H-azepino-[1,2-a]-indol-8(RS)-yl]-2-methylpropyl]carbamate there were obtained 350 mg of 3-[8(RS)-[1(RS)-tert.butoxyformamido-2-methylpropyl]-7,8,9,10-tetrahydro-6H-azepino[1,2-a]indol-11-yl]-4-(1-methyl-3-indolyl)-1H-pyrrole-2,5-dione as a red oil.

Example 20

In a manner analogous to that described in the first paragraph of Example 1, from 800 mg of 3-[7(RS)-[1(RS)-tert.butoxyformamido-2-methylpropyl]-6,7,8,9-tetrahydropyrido[1,2-a]indol-10-yl]-4-(1-methyl-3-indolyl)-1H-pyrrole-2,5-dione there were obtained 480 mg of 3-[7(RS)-[1(RS)-amino-2-methylpropyl]-6,7,8,9-tetrahydropyrido[1,2-a]indol-10-yl]-4-(1-methyl-3-indolyl)-1H-pyrrole-2,5-dione hydrochloride as a red solid of melting point 238-244°C.

The 3-[7(RS)-[1(RS)-tert.butoxyformamido-2-methylpropyl]-6,7,8,9-tetrahydropyrido[1,2-a]indol-10-yl]-4-(1-methyl-3-indolyl)-1H-pyrrole-2,5-dione used as the starting material was prepared as follows:

(i) In a manner analogous to that described in Example 11(ii), from 2.0 g (9.3 mmol) of 6,7,8,9-tetrahydropyrido[1,2-a]indole-7(RS)-carboxylic acid there were obtained 1.6 g of 6,7,8,9-tetrahydro-N-methoxy-N-methyl-pyrido[1,2-a]indole-7(RS)-carboxamide as a pale yellow oil.

(ii) In a manner analogous to that described in Example 17(iii), from 1.6 g of 6,7,8,9-tetrahydro-N-methoxy-N-methyl-pyrido[1,2-a]-indole-7(RS)-carboxamide there were obtained 1.05 g of isopropyl 6,7,8,9-tetrahydropyrido[1,2-a]indol-7(RS)-yl ketone as a tan solid of melting point 43-44°C.

(iii) In a manner analogous to that described in Example 11(iv), from 1.0 g of isopropyl 6,7,8,9-tetrahydropyrido[1,2-a]indol-7(RS)-yl ketone there were obtained 800 mg of tert.butyl [1(RS)-[6,7,8,9-tetrahydropyrido[1,2-a]indol-7(RS)-yl]-2-methylpropyl]carbamate as a white solid of melting point 55-57°C (as a mixture of diastereomers).

(iv) In a manner analogous to that described in Example 1(iv), from 700 mg of tert.butyl [1(RS)-[6,7,8,9-tetrahydropyrido[1,2-a]indol-7(RS)-yl]-2-methylpropyl]carbamate there were obtained 800 mg of 3-[7(RS)-[1(RS)-tert.butoxyformamido-2-methylpropyl]-6,7,8,9-tetrahydropyrido[1,2-a]indol-10-yl]-4-(1-methyl-3-indolyl)-1H-pyrrole-2,5-dione as a red gum.

Example 21

In a manner analogous to that described in the first paragraph of Example 1, from 1.3 g of 3-[8(S)-[1(S)-tert.butoxyformamido-2-methylpropyl]-6,7,8,9-tetrahydropyrido[1,2-a]indol-10-yl]-4-(1-phenyl-3-indolyl)-1H-pyrrole-2,5-dione there were obtained 1.12 g of 3[8(S)-[1(S)-amino-2-methylpropyl]-6,7,8,9-tetrahydropyrido[1,2-a]indol-10-yl]-4-(1-phenyl-3-indolyl)-1H-pyrrole-2,5-dione hydrochloride as a red solid of melting point 235-245°C.

The 3-[8(S)-[1(S)-tert.butoxyformamido-2-methylpropyl]-6,7,8,9-tetrahydropyrido[1,2-a]indol-10-yl]-4-(1-phenyl-3-indolyl)-1H-pyrrole-2,5-dione used as the starting material was prepared as follows:

(i) An ice-cooled solution of 10 g (51.8 mmol) of 1-phenylindole in 100 ml of anhydrous diethyl ether was treated dropwise during 5 minutes with a solution of 6 ml (68.8 mmol) of oxalyl chloride in 20 ml of anhydrous diethyl ether. The mixture was stirred for 3 hours while cooling with ice and then treated with 25 ml of ethanol in one portion. After stirring for 10 minutes the solvent was removed under reduced pressure and the residual solid was crystallized from 60 ml of ethanol to give 12.38 g of ethyl 1-phenylindole-3-glyoxylate as a pale yellow solid of melting point 109-110°C.

(ii) A mixture of 10 g (34.1 mmol) of ethyl 1-phenylindole-3-glyoxylate and about 25 g of Raney nickel in 350 ml of ethanol and 150 ml of water was heated at reflux for 6 hours. The suspension was filtered through glass fibre filter paper and the solid was washed with four 50 ml portions of ethyl acetate while taking care not to allow the solid to dry. The filtrate was concentrated under reduced pressure and the residue was purified by flash chromatography on silica gel using ethyl acetate/hexane (1:2) for the elution. There were obtained 6.38 g of ethyl 1-phenylindole-3-acetate as a yellow oil.

(iii) A solution of 6.3 g (22.6 mmol) of ethyl 1-phenylindole-3-acetate in 20 ml of ethanol was treated with 20 ml (40 mmol) of 2M sodium hydroxide solution and left at room temperature for 17 hours. Ethanol was removed under reduced pressure and the aqueous solution was washed with two 20 ml portions of diethyl ether. The aqueous phase was acidified with concentrated hydrochloric acid and the suspension obtained was stored at 0°C for 2 hours. The suspension was filtered and the solid was crystallized from methanol/water (2:1) to give 5.6 g 1-phenylindole-3-acetic acid as a blue-grey solid of melting point 131-135°C.

(iv) An ice-cooled solution of 3 g (8.77 mmol) of tert.butyl [1(S)-[6,7,8,9-tetrahydropyrido[1,2-a]indol-8(S)-yl]-2-methylpropyl]carbamate, prepared as described in Example 11(iv), in 50 ml of anhydrous diethyl ether was treated dropwise under a nitrogen atmosphere over a period of 5 minutes with a solution of 0.85 ml (9.74 mmol) of oxalyl chloride in 5 ml of anhydrous diethyl ether. After a further 5 minutes the solvent was removed under reduced pressure and the residue was dissolved in 50 ml of dry dichloromethane. The solution was added dropwise at 0°C to a stirred mixture of 2.2 g (8.77 mmol) of 1-phenylindole-3-acetic acid and 3.65 ml (26.3 mmol) of triethylamine in 50 ml of dry dichloromethane. The mixture was stirred for 17 hours then the solvent was removed under reduced pressure. Purification by flash chromatography on silica gel using ethyl acetate/hexane (1:2) for the elution followed by crystallization from ethyl acetate/hexane gave 1.6 g of 3[8(S)-[1(S)-tert.butoxyformamido-2-methylpropyl]-6,7,8,9-tetrahydropyrido[1,2-a]indol-10-yl]-4-(1-phenyl-3-indolyl)-furan-2,5-dione as an orange coloured solid of melting point 148-150°C.

(v) A solution of 1.6 g (2.54 mmol) of 3[8(S)-[1(S)-tert.-butoxyformamido-2-methylpropyl]-6,7,8,9-tetrahydropyrido[1,2-a]indol-10-yl]-4-(1-phenyl-3-indolyl)-furan-2,5-dione in 20 ml of dry N,N-dimethylformamide was treated with 5.35 ml (25.4 mmol) of hexamethyldisilazane and 0.41 g (12.8 mmol) of methanol. The solution was heated at 50°C for 3 hours and then treated with a further 5.35 ml (24.8 mmol) of hexamethyldisilazane and 0.41 g (12.8 mmol) of methanol. After a total of 6 hours the solvent was evaporated under reduced pressure and the residue was re-evaporated with 20 ml of methanol. Flash chromatography of the residue on silica gel using ethyl acetate/hexane (1:2) for the elution gave 1.35 g of 3[8(S)-[1(S)-tert.butoxyformamido-2-methylpropyl]-6,7,8,9-tetrahydropyrido[1,2-a]indol-10-yl]-4-(1-phenyl-3-indolyl)-1H-pyrrole-2,5-dione as a red solid of melting point 165-168°C.

The following Examples illustrate typical pharmaceutical preparations containing compounds provided by the present invention:

Example A

Tablets containing the following ingredients may be produced in a conventional manner:

| Ingredient | Per tablet |
|---|---|
| Compound of formula I | 5.0 mg |
| Lactose | 125.0 mg |
| Maize starch | 75.0 mg |
| Talc | 4.0 mg |
| Magnesium stearate | 1.0 mg |
| Tablet weight | 210.0 mg |

Example B

Capsules containing the following ingredients may be produced in a conventional manner:

| Ingredient | Per capsule |
|---|---|
| Compound of formula I | 10.0 mg |
| Lactose | 165.0 mg |
| Maize starch | 20.0 mg |
| Talc | 5.0 mg |
| Capsule fill weight | 200.0 mg |

## Claims

1. Compounds of the general formula I

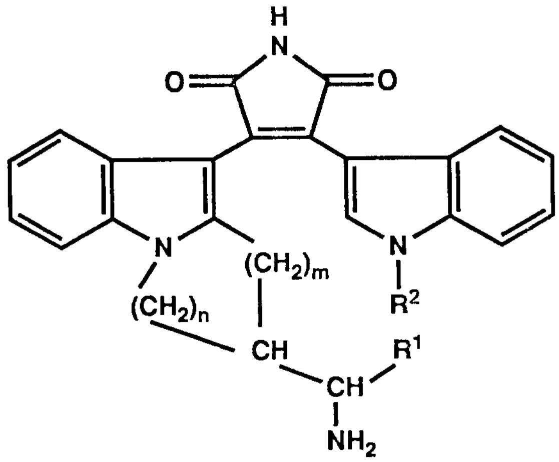

I

wherein $R^1$ represents lower alkyl, lower cycloalkyl, aryl or lower aralkyl, $R^2$ represents hydrogen, aryl or lower alkyl optionally substituted by hydroxy, acyloxy, amino, mono(lower alkyl)amino, di(lower alkyl)amino, carboxy, lower alkoxycarbonyl or aminocarbonyl and m and n stand for 1 or 2,
as well as pharmaceutically acceptable salts of acidic compounds of formula I with bases and of basic compounds of formula I with acids;
wherein "lower alkyl" means a straight-chain or branched-chain alkyl group containing 1-6 carbon atoms, "lower cycloalkyl" means a cycloalkyl group containing 3-6 carbon atoms, "lower alkoxy" means an alkyl group as defined before which is attached via an oxygen atom, "aryl" means unsubstituted phenyl or phenyl carrying one or more substituents selected from halogen, lower alkyl and lower alkoxy as defined before, "lower aralkyl" means a lower alkyl group as defined before in which one hydrogen atom has been replaced by an aryl group as defined before, "acyloxy" means an acyloxy group derived from an alkanoic acid containing up to 6 carbon atoms or from an aromatic carboxylic acid which can be optionally substituted by halogen, lower alkyl and/or lower alkoxy as defined before.

2. Compounds according to claim 1, wherein $R^1$ represents lower alkyl containing 1-6 carbon atoms.

3. Compounds according to claim 1, wherein $R^1$ represents lower alkyl containing 1-3 carbon atoms.

4. Compounds according to any one of claims 1-3, wherein $R^2$ represents lower alkyl containing 1-6 carbon atoms.

5. Compounds according to any one of claims 1-4, wherein m stands for 1 and n stands for 2.

6. A compound according to claim 1 having the formula:

   3-[8(S)-[1(R or S)-Aminopropyl]-6,7,8,9-tetrahydropyrido[1,2-a]indol-10-yl]-4-(1-methyl-3-indolyl)-1H-pyrrole-2,5-dione.

7. A compound according to claim 1 having the formula:

   3-[8(S)-[1(S)-Amino-2-methylpropyl]-6,7,8,9-tetrahydropyrido[1,2-a]indol-10-yl]-4-(1-methyl-3-indolyl)-1H-pyrrole-2,5-dione.

8. A compound according to claim 1 selected from the following:

   3-[8(R or S)-1(R or S)-Aminoethyl]-6,7,8,9-tetrahydropyrido[1,2-a]indol-10-yl]-4-(1-methyl-3-indolyl)-1H-pyrrole-2,5-dione,
   3-[8(R or S)-1(R or S)-aminopropyl]-6,7,8,9-tetrahydropyrido[1,2-a]indol-10-yl]-4-(1-methyl-3-indolyl)-1H-pyrrole-2,5-dione,
   3-[8(R or S)-1(R or S)-aminobutyl]-6,7,8,9-tetrahydropyrido[1,2-a]indol-10-yl]-4-(1-methyl-3-indolyl)-1H-pyrrole-2,5-dione and
3-[8(R or S)-1(R or S)-amino-2-methylpropyl]-6,7,8,9-tetrahydropyrido[1,2-a]indol-10-yl]-4-(1-methyl-3-indolyl)-1H-pyrrole-2,5-dione.

**9.** A compound according to claim 1, selected from the following:

3-[8(S)-1(R)-amino-2-methylpropyl]-6,7,8,9-tetrahydropyrido[1,2-a]indol-10-yl]-4-(1-methyl-3-indolyl)-1H-pyrrole-2,5-dione,
3-[8(R or S)-[alpha (R or S)-aminobenzyl]-6,7,8,9-tetrahydropyrido[1,2-a]indol-10-yl]-4-(1-methyl-3-indolyl)-1H-pyrrole-2,5-dione,
3-[8(S)-[(R or S)-(amino)(cyclopentyl)methyl-6,7,8,9-tetrahydropyrido[1,2-a]indol-10-yl]-4-(1-methyl-3-indolyl)-1H-pyrrole-2,5-dione,
3-[2(R or S)-[1(R or S)-amino-2-methylpropyl]-2,3-dihydro-1H-pyrrolo[1,2-a]indol-9-yl]-4-(1-methyl-3-indolyl)-1H-pyrrole-2,5-dione,
3-[8(RS)-[1(RS)-amino-2-methylpropyl]-7,8,9,10-tetrahydro-6H-azepino[1,2-a]indol-11-yl]-4-(1-methyl-3-indolyl)-1H-pyrrole-2,5-dione,
3-[7(RS)-[1(RS)-amino-2-methylpropyl]-6,7,8,9-tetrahydropyrido[1,2-a]indol-9-yl]-4-(1-methyl-3-indolyl)-1H-pyrrole-2,5-dione and
3-[8(S)-[1(S)-amino-2-methylpropyl]-6,7,8,9-tetrahydropyrido[1,2-a]indol-10-yl]-4-(1-phenyl-3-indolyl)-1H-pyrrole-2,5-dione.

**10.** Compounds of the general formula

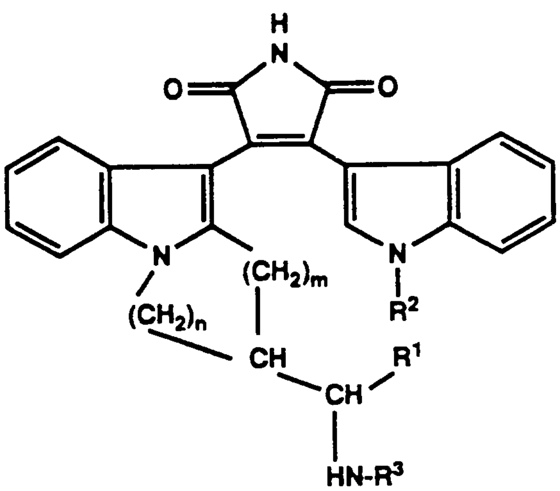

II

wherein $R^1$, $R^2$, m and n have the significance given in claim 1 and $R^3$ represents a urethane protecting group.

**11.** Compounds according to any one of claims 1 to 9 for use as therapeutically active substances, particularly for use as antiinflammatory, immunological, oncological, broncho-pulmonary, dermatological and cardiovascular active substances, as active substances in the treatment of asthma, AIDS or diabetic complications or as active substances for the stimulation of hair growth.

**12.** A process for the manufacture of the compounds according to any one of claims 1 to 9 which process comprises cleaving off the protecting group denoted by $R^3$ from a compound of the general formula

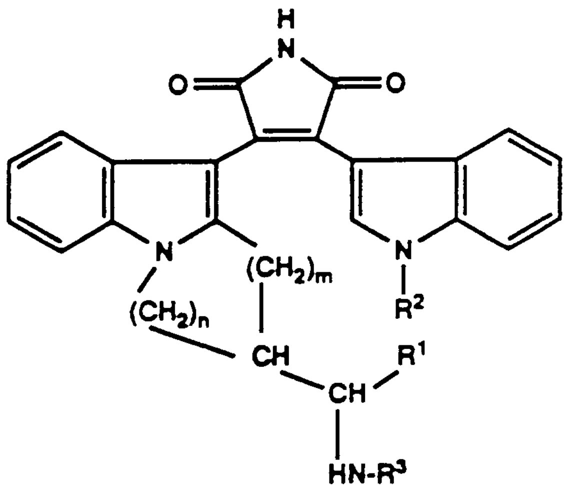

II

wherein $R^1$, $R^2$, m and n have the significance given in claim 1 and $R^3$ represents a urethane protecting group,
and, if desired, functionally modifying a reactive substituent present in $R^2$ in a compound of formula I obtained and, also if desired, converting an acidic compound of formula I into a pharmaceutically acceptable salt with a base or converting a basic compound of formula I into a pharmaceutically acceptable salt with an acid.

**13.** A medicament, particularly an antiinflammatory, immunological, oncological, bronchopulmonary, dermatological or cardiovascular medicament or a medicament for the treatment of asthma, AIDS or diabetic complications or for the stimulation of hair growth, containing a compound according to any one of claims 1 to 9 and a therapeutically inert carrier material.

**14.** The use of a compound according to any one of claims 1 to 9 for the manufacture of a medicament against inflammatory, immunological, oncological, bronchopulmonary, dermatological or cardiovascular disorders or against asthma, AIDS or diabetic complications or for the stimulation of hair growth.

## Patentansprüche

**1.** Verbindungen der allgemeinen Formel I

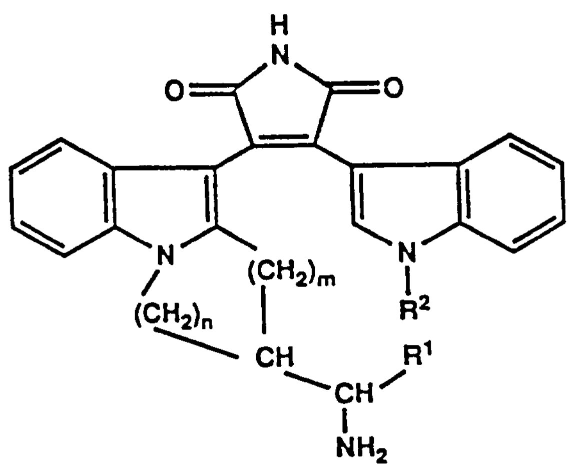

I

in der $R^1$ einen Niederalkyl-, Niedercycloalkyl-, Aryl- oder Niederaralkylrest wiedergibt, $R^2$ ein Wasserstoffatom, einen Aryl- oder einen Niederalkylrest wiedergibt, der gegebenenfalls mit einer Hydroxy-, Acyloxy-, Amino-, Mono-(Niederalkyl)amino-, Di-(Niederalkyl)amino-, Carboxy-, Niederalkoxycarbonyl- oder Aminocarbonylgruppe substituiert ist, und m und n die Bedeutung 1 oder 2 haben,
sowie pharmazeutisch verträgliche Salze saurer Verbindungen der Formel I mit Basen und basischen Verbindungen der Formel I mit Säuren;
wobei "Niederalkylrest" einen geradkettigen oder verzweigtkettigen Alkylrest mit 1-6 Kohlenstoffatomen bedeutet, "Niedercycloalkylrest" einen Cycloalkylrest mit 3-6 Kohlenstoffatomen bedeutet, "Niederalkoxyrest" einen Alkylrest, wie vorstehend definiert, bedeutet, der über ein Sauerstoffatom gebunden ist, "Arylrest" eine unsubstituierte Phenylgruppe oder eine Phenylgruppe bedeutet, die einen oder mehrere Substituenten trägt, ausgewählt aus Halogenatomen, Niederalkyl- und Niederalkoxyresten, wie vorstehend definiert, "Niederaralkylrest" einen Niederalkylrest, wie vorstehend definiert, bedeutet, bei dem ein Wassestoffatom durch einen Arylrest, wie vorstehend definiert, ersetzt ist, "Acyloxyrest" einen Acyloxyrest bedeutet, der von einer Alkansäure mit bis zu 6 Kohlenstoffatomen oder von einer aromatischen Carbonsäure abstammt, die gegebenenfalls mit einem Halogenatom, einem Niederalkyl- und/oder Niederalkoxyrest, wie vorstehend definiert, substituiert sein kann.

**2.** Verbindungen nach Anspruch 1, wobei $R^1$ einen Niederalkylrest mit 1-6 Kohlenstoffatomen wiedergibt.

**3.** Verbindungen nach Anspruch 1, wobei $R^1$ einen Niederalkylrest mit 1-3 Kohlenstoffatomen wiedergibt.

**4.** Verbindungen nach einem der Ansprüche 1-3, wobei $R^2$ einen Niederalkylrest mit 1-6 Kohlenstoffatomen wiedergibt.

**5.** Verbindungen nach einem der Ansprüche 1-4, wobei m die Bedeutung 1 hat und n die Bedeutung 2 hat.

**6.** Verbindung nach Anspruch 1 mit der Formel:

3-[8(S)-[1(R oder S)-Aminopropyl]-6,7,8,9-tetrahydropyrido-[1,2-a]indol-10-yl]-4-(1-methyl-3-indolyl)-1H-pyrrol-2,5-dion.

**7.** Verbindung nach Anspruch 1 mit der Formel:

3-[8(S)-[1(S)-Amino-2-methylpropyl]-6,7,8,9-tetrahydropyrido[1,2-a]indol-10-yl]-4-(1-methyl-3-indolyl)-1H-pyrrol-2,5-dion.

**8.** Verbindung nach Anspruch 1, ausgewählt aus den nachstehenden:

3-[8(R oder S)-1(R oder S)-Aminoethyl]-6,7,8,9-tetrahydropyrido[1,2-a]indol-10-yl]-4-(1-methyl-3-indolyl)-1H-pyrrol-2,5-dion,
3-[8(R oder S)-1(R oder S)-Aminopropyl]-6,7,8,9-tetrahydropyrido[1,2-a]indol-10-yl]-4-(1-methyl-3-indolyl)-1H-pyrrol-2,5-dion,
3-[8(R oder S)-1(R oder S)-Aminobutyl]-6,7,8,9-tetrahydropyrido[1,2-a]indol-10-yl]-4-(1-methyl-3-indolyl)-1H-pyrrol-2,5-dion und
3-[8(R oder S)-1(R oder S)-Amino-2-methylpropyl]-6,7,8,9-tetrahydropyrido[1,2-a]indol-10-yl]-4-(1-methyl-3-indolyl)-1H-pyrrol-2,5-dion.

**9.** Verbindung nach Anspruch 1, ausgewählt aus den nachstehenden:

3-[8(S)-1(R)-Amino-2-methylpropyl]-6,7,8,9-tetrahydropyrido[1,2-a]indol-10-yl]-4-(1-methyl-3-indolyl)-1H-pyrrol-2,5-dion,
3-[8(R oder S)-[alpha (R oder S)-Aminobenzyl]-6,7,8,9-tetrahydropyrido[1,2-a]indol-10-yl]-4-(1-methyl-3-indolyl)-1H-pyrrol-2,5-dion,
3-[8(S)-[(R oder S)-(Amino)(cyclopentyl)methyl-6,7,8,9-tetrahydropyrido[1,2-a]indol-10-yl]-4-(1-methyl-3-indolyl)-1H-pyrrol-2,5-dion,
3-[2(R oder S)-[1(R oder S)-Amino-2-methylpropyl]-2,3-dihydro-1H-pyrrolo[1,2-a]indol-9-yl]-4-(1-methyl-3-indolyl)-1H-pyrrol-2, 5-dion,
3-[8(RS)-[1(RS)-Amino-2-methylpropyl]-7,8,9,10-tetrahydro-6H-azepino[1,2-a]indol-11-yl]-4-(1-methyl-3-indolyl)-1H-pyrrol-2,5-dion,
3-[7(RS)-[1(RS)-Amino-2-methylpropyl]-6,7,8,9-tetrahydropyrido[1,2-a]indol-9-yl]-4-(1-methyl-3-indolyl)-1H-pyrrol-2,5-dion und

3-[8(S)-[1(S)-Amino-2-methylpropyl]-6,7,8,9-tetrahydropyrido[1,2-a]indol-10-yl]-4-(1-phenyl-3-indolyl)-1H-pyrrol-2,5-dion.

**10.** Verbindungen der allgemeinen Formel

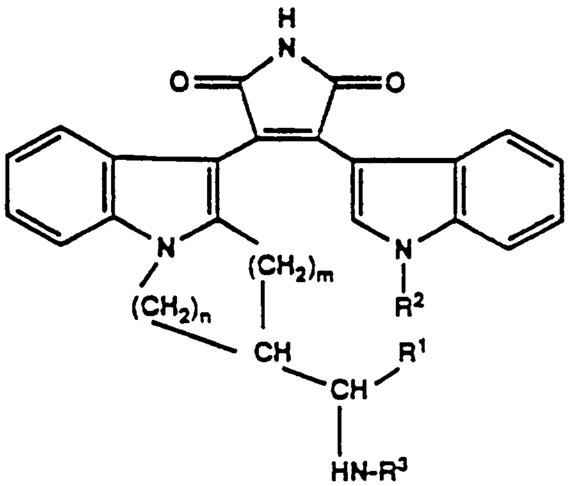

II

in der $R^1$, $R^2$, m und n die im Anspruch 1 angegebene Bedeutung haben und $R^3$ eine Urethanschutzgruppe wiedergibt.

**11.** Verbindungen nach einem der Ansprüche 1 bis 9 zur Verwendung als therapeutisch wirksame Substanzen, insbesondere zur Verwendung als entzündungshemmende, immunologisch, onkologisch, bronchopulmonal, dermatologisch und kardiovaskulär wirksame Substanzen, als wirksame Substanzen zur Behandlung von Asthma, AIDS oder Diabeteskomplikationen oder als wirksame Substanzen zur Stimulation des Haarwuchses.

**12.** Verfahren zur Herstellung der Verbindungen nach einem der Ansprüche 1 bis 9, wobei das Verfahren das Abspalten der durch $R^3$ wiedergegebenen Schutzgruppe von einer Verbindung der allgemeinen Formel

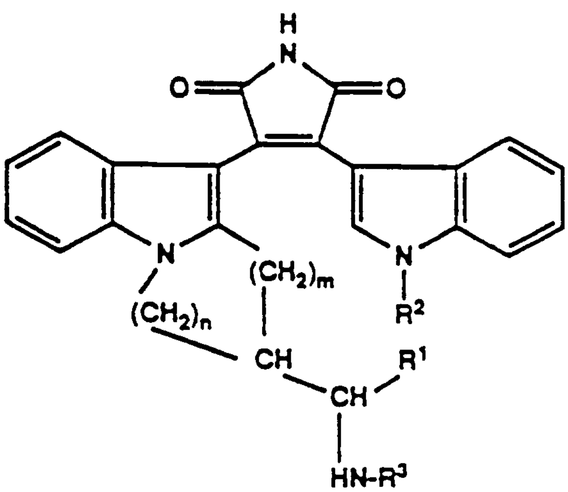

II

in der $R^1$, $R^2$, m und n die im Anspruch 1 angegebene Bedeutung haben und $R^3$ eine Urethanschutzgruppe wiedergibt,
und, wenn erwünscht, das funktionelle Modifizieren eines reaktiven Substituenten, der sich im Rest $R^2$ einer erhaltenen Verbindung der Formel I befindet, und, ebenfalls wenn erwünscht, das Umwandeln einer sauren Verbindung der Formel I in ein pharmazeutisch verträgliches Salz mit einer Base oder das Umwandeln einer basischen Verbindung der Formel I in ein pharmazeutisch verträgliches Salz mit einer Säure umfaßt.

13. Medikament, insbesondere ein entzündungshemmendes, immunologisches, onkologisches, bronchopulmonales, dermatologisches oder kardiovaskuläres Medikament oder ein Medikament zur Behandlung von Asthma, AIDS oder Diabeteskomplikationen oder zur Stimulation des Haarwuchses, das eine Verbindung nach einem der Ansprüche 1 bis 9 und ein therapeutisch inertes Trägermaterial enthält.

14. Verwendung einer Verbindung nach einem der Ansprüche 1 bis 9 zur Herstellung eines Medikamentes gegen entzündliche, immunologische, onkologische, bronchopulmonale, dermatologische oder kardiovaskuläre Erkrankungen oder gegen Asthma, AIDS oder Diabeteskomplikationen oder zur Stimulation des Haarwuchses.

## Revendications

1. Composés de formule générale I:

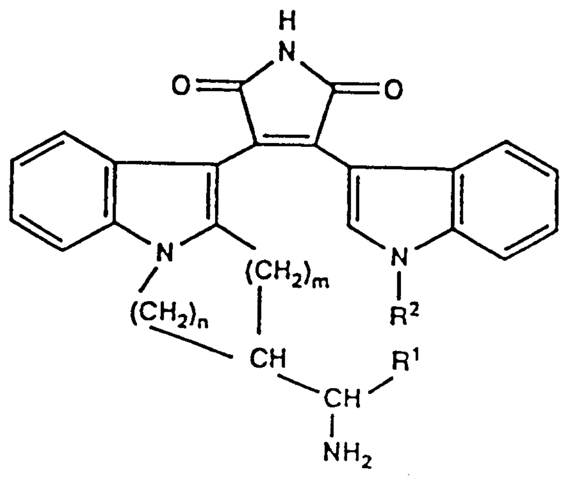

I

dans laquelle $R^1$ représente un groupe alkyle inférieur, cycloalkyle inférieur, aryle ou aralkyle inférieur, $R^2$ représente un atome d'hydrogène, un groupe aryle ou alkyle inférieur éventuellement substitué par un groupe hydroxy, acyloxy, amino, mono(alkyle inférieur)amino, di(alkyle inférieur)amino, carboxy, alcoxycarbonyle inférieur ou aminocarbonyle et m et n valent 1 ou 2,
ainsi que les sels acceptables sur le plan pharmaceutique de composés acides de formule I avec des bases et de composés basiques de formule I avec des acides;
dans lesquels "alkyle inférieur" signifie un groupe alkyle à chaîne droite ou ramifiée contenant 1 à 6 atomes de carbone, "cycloalkyle inférieur" signifie un groupe cycloalkyle contenant 3 à 6 atomes de carbone, "alcoxy inférieur" signifie un groupe alkyle comme défini précédemment qui est fixé par l'intermédiaire d'un atome d'oxygène, "aryle" signifie un groupe phényle non substitué ou un phényle portant un ou plusieurs substituants choisis parmi un atome d'halogène, un groupe alkyle inférieur et alcoxy inférieur comme défini précédemment, "aralkyle inférieur" signifie un groupe alkyle inférieur comme défini précédemment dans lequel un atome d'hydrogène a été remplacé par un groupe aryle comme défini précédemment, "acyloxy" signifie un groupe acyloxy dérivé d'un acide alcanoïque contenant jusqu'à 6 atomes de carbone ou d'un acide carboxylique aromatique qui peut éventuellement être substitué par un atome d'halogène, un groupe alkyle inférieur et/ou alcoxy inférieur comme défini précédemment.

2. Composés selon la revendication 1, dans lesquels $R^1$ représente un groupe alkyle inférieur contenant 1 à 6 atomes de carbone.

3. Composés selon la revendication 1, dans lesquels $R^1$ représente un groupe alkyle inférieur contenant 1 à 3 atomes de carbone.

4. Composés selon l'une quelconque des revendications 1 à 3, dans lesquels $R^2$ représente un groupe alkyle, contenant 1 à 6 atomes de carbone.

5. Composés selon l'une quelconque des revendications 1 à 4, dans lesquels m vaut 1 et n vaut 2.

**6.** Composé selon la revendication 1, ayant la formule:

3-[8(S)-[1(R ou S)-aminopropyl]-6,7,8,9-tétrahydropyrido[1,2-a]indol-10-yl]-4-(1-méthyl-3-indolyl)-1H-pyrrole-2,5-dione.

**7.** Composé selon la revendication 1, ayant la formule:

3-[8(S)-[1(S)-amino-2-méthylpropyl]-6,7,8,9-tétrahydropyrido[1,2-a]indol-10-yl]-4-(1-méthyl-3-indolyl)-1H-pyrrole-2,5-dione.

**8.** Composé selon la revendication 1, choisi parmi ce qui suit:

- la 3-[8(R ou S)-1(R ou S)-aminoéthyl]-6,7,8,9-tétrahydropyrido[1,2-a]indol-10-yl]-4-(1-méthyl-3-indolyl)-1H-pyrrole-2,5-dione;
- la 3-[8(R ou S)-1(R ou S)-aminopropyl]-6,7,8,9-tétrahydropyrido[1,2-a]indol-10-yl]-4-(1-méthyl-3-indolyl)-1H-pyrrole-2,5-dione;
- la 3-[8(R ou S)-1(R ou S)-aminobutyl]-6,7,8,9-tétrahydropyrido[1,2-a]indol-10-yl]-4-(1-méthyl-3-indolyl)-1H-pyrrole-2,5-dione; et
- la 3-[8(R ou S)-1(R ou S)-amino-2-méthylpropyl]-6,7,8,9-tétrahydropyrido[1,2-a]indol-10-yl]-4-(1-méthyl-3-indolyl)-1H-pyrrole-2,5-dione.

**9.** Composé selon la revendication 1, choisi dans ce qui suit:

- la 3-[8(S)-1(R)-amino-2-méthylpropyl]-6,7,8,9-tétrahydropyrido[1,2-a]indol-10-yl]-4-(1-méthyl-3-indolyl)-1H-pyrrole-2,5-dione;
- la 3-[8(R ou S)-[alpha(R ou S)-aminobenzyl]-6,7,8,9-tétrahydropyrido[1,2-a]indol-10-yl]-4-(1-méthyl-3-indolyl)-1H-pyrrole-2,5-dione;
- la 3-[8(S)-[(RouS)-(amino)(cyclopentyl)méthyl-6,7,8,9-tétrahydropyrido[1,2-a]indol-10-yl]-4-(1-méthyl-3-indolyl)-1H-pyrrole-2,5-dione;
- la 3-[2(R ou S)-[1(R ou S)-amino-2-méthylpropyl]-2,3-dihydro-1H-pyrrolo[1,2-a]indol-9-yl]-4-(1-méthyl-3-indolyl)-1H-pyrrole-2,5-dione;
- la 3-[8(RS)-[1(RS)-amino-2-méthylpropyl]-7,8,9,10-tétrahydro-6H-azépino[1,2-a]indol-11-yl]-4-(1-méthyl-3-indolyl)-1H-pyrrole-2,5-dione;
- la 3-[7(RS)-[1(RS)-amino-2-méthylpropyl]-6,7,8,9-tétrahydropyrido[1,2-a]indol-9-yl]-4-(1-méthyl-3-indolyl)-1H-pyrrole-2,5-dione; et
- la 3-[8(S)-[1(S)-amino-2-méthylpropyl]-6,7,8,9-tétrahydropyrido[1,2-a]indol-10-yl]-4-(1-phényl-3-indolyl)-1H-pyrrole-2,5-dione.

**10.** Composés de formule générale:

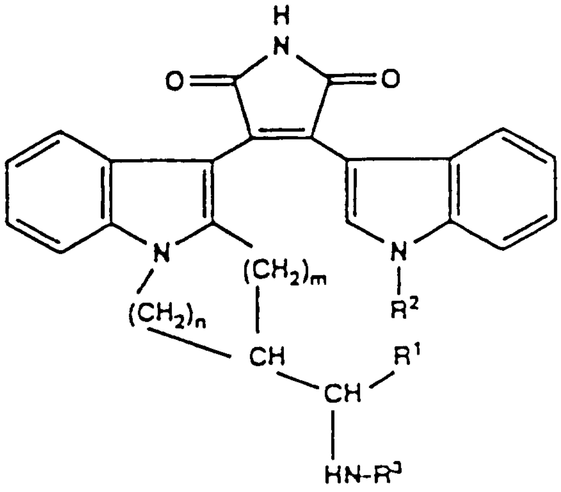

II

dans laquelle $R^1$, $R^2$, m et n ont la signification donnée dans la revendication 1 et $R^3$ représente un groupe de protection uréthane.

**11.** Composés selon l'une quelconque des revendications 1 à 9, pour une utilisation en tant que substances thérapeutiquement actives, en particulier pour une utilisation en tant que substances actives antiinflammatoires, immunologiques, oncologiques, broncho-pulmonaires, dermatologiques et cardiovasculaires, en tant que substances actives dans le traitement de l'asthme, du SIDA ou des complications diabétiques ou en tant que substances actives pour la stimulation de la pousse des cheveux.

**12.** Procédé pour la fabrication des composés selon l'une quelconque des revendications 1 à 9, procédé qui consiste à couper le groupe de protection indiqué par $R^3$ d'avec un composé de formule générale:

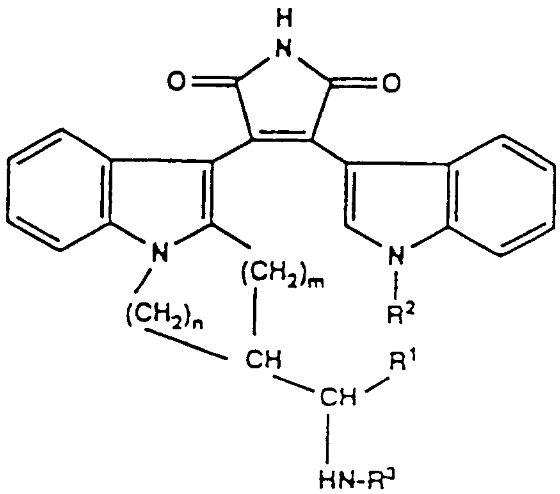

II

dans laquelle $R^1$, $R^2$, m et n ont la signification donnée dans la revendication 1 et $R^3$ représente un groupe de protection uréthane,
et, si souhaité, à modifier fonctionnellement un substituant réactif présent dans $R^2$ dans un composé de formule I obtenu et, également si souhaité, à convertir un composé acide de formule I en un sel acceptable sur le plan pharmaceutique avec une base ou à convertir un composé basique de formule I en un sel acceptable sur le plan pharmaceutique avec un acide.

**13.** Médicament, en particulier un médicament antiinflammatoire, immunologique, oncologique, broncho-pulmonaire, dermatologique ou cardiovasculaire ou un médicament pour le traitement de l'asthme, du SIDA ou des complications diabétiques ou pour la stimulation de la pousse des cheveux, contenant un composé selon l'une quelconque des revendications 1 à 9 et une matière véhicule thérapeutiquement inerte.

**14.** Utilisation d'un composé selon l'une quelconque des revendications 1 à 9, pour la fabrication d'un médicament contre les troubles inflammatoires, immunologiques, oncologiques, broncho-pulmonaires, dermatologiques ou cardiovasculaires ou contre l'asthme, le SIDA ou des complications diabétiques ou pour la stimulation de la pousse des cheveux.